# EUROPEAN PATENT SPECIFICATION

(11) **EP 3 347 098 B1**
(45) Date of publication and mention of the grant of the patent: **05.01.2022**
(21) Application number: 16845100.3
(22) Date of filing: 09.09.2016
(51) Int. Cl.: A61P 35/00, C12N 5/10, A61K 35/76, C12N 15/861

(54) **TARGETING MDA-5 ACTIVATION FOR CANCER IMMUNOTHERAPY**
ABZIELUNG AUF MDA-5-AKTIVIERUNG FÜR KREBSIMMUNTHERAPIE
CIBLAGE DE L'ACTIVATION DE MDA-5 POUR L'IMMUNOTHÉRAPIE DU CANCER

(30) Priority: 09.09.2015 US 201562215887 P
(43) Date of publication of application: 18.07.2018
(73) Proprietor: Virginia Commonwealth University, Richmond, VA 23298 (US)
(72) Inventor: WANG, Xiang-yang, Glen Allen, VA 23059 (US); FISHER, Paul, B., Henrico, VA 23238 (US); YU, Xiaofei, Richmond, VA 23229 (US)
(74) Representative: Gramm, Lins & Partner Patent- und Rechtsanwälte PartGmbB
(86) International application number: PCT/US2016/050900
(87) International publication number: WO 2017/044709

(56) References cited:
- US-A1- 2008 213 220
- XIAOFEI YU ET AL: "Inhibition of tumor growth by targeting the MDA5-IPS-1 pathway requires coordinated induction of cancer cell death and immune activation (TUM2P.891)", J. IMMUNOL., vol. 21, no. S1, 1 January 2014 (2014-01-01), page 71.15, XP055589278,
- ROBERT BESCH ET AL: "Proapoptotic signaling induced by RIG-I and MDA-5 results in type I interferon-independent apoptosis in human melanoma cells", JOURNAL OF CLINICAL INVESTIGATION, 1 January 2009 (2009-01-01), XP055589322, GB ISSN: 0021-9738, DOI: 10.1172/JCI37155
- X. YU ET AL: "A Multifunctional Chimeric Chaperone Serves as a Novel Immune Modulator Inducing Therapeutic Antitumor Immunity", CANCER RESEARCH, vol. 73, no. 7, 18 January 2013 (2013-01-18), pages 2093-2103, XP055589282, Proceedings: AACR Annual Meeting 2014; April 5-9, 2014; San Diego, CA ISSN: 0008-5472, DOI: 10.1158/0008-5472.CAN-12-1740
- SIDDIK SARKAR ET AL: "Therapy of prostate cancer using a novel cancer terminator virus and a small molecule BH-3 mimetic", ONCOTARGET, vol. 6, no. 13, 12 March 2015 (2015-03-12) , pages 10712-10727, XP055323756, DOI: 10.18632/oncotarget.3544
- DREW M. PARDOLL: "The blockade of immune checkpoints in cancer immunotherapy", NATURE REVIEWS. CANCER, vol. 12, no. 4, 1 April 2012 (2012-04-01), pages 252-264, XP055415943, GB ISSN: 1474-175X, DOI: 10.1038/nrc3239
- ZITVOGEL ET AL.: 'Anticancer immunochemotherapy using adjuvants with direct cytotoxic effects.' J CLIN INVEST vol. 119, no. 8, August 2009, pages 2127 - 2130, XP055372726
- KANG ET AL.: 'mda-5: An interferon-inducible putative RNA helicase with double-stranded RNA- dependent ATPase activity and melanoma growth-suppressive properties.' PROC NAT ACAD SCI vol. 99, no. 2, 22 January 2002, pages 637 - 642, XP003018262
- YU ET AL.: 'Activation of the MDA-5-IPS-1 Viral Sensing Pathway Induces Cancer Cell Death and Type I IFN-Dependent Antitumor Immunity.' CANCER RES vol. 76, no. 8, 18 February 2016, pages 2166 - 2176, XP055372729

## Description

### FIELD OF THE INVENTION

The invention generally relates to the treatment and prevention of the recurrence of cancer by expression of nucleic acids encoding MDA-5 or functional conservative derivatives thereof. The therapeutic agent for use according to the invention induces regression of pre-established cancers and development of long-lasting antitumor immune memory

### BACKGROUND OF THE INVENTION

Cancer is one of the leading causes of death and is responsible for increasing health costs. Traditionally, cancer has been treated using chemotherapy, radiotherapy and surgical methods. Tumor cell plasticity and heterogeneity, however, remain challenges for effective treatments of many cancers. In addition, traditional therapies may have drawbacks, e.g. insufficient specificity, intolerable toxicity and too low efficacy.

In recent years molecular therapies have been developed, which eliminate cancer cells and prolong the survival time of affected patients or cure said patients. One area of interest in the field of cancer therapy is directed to the induction of apoptosis in cancer cells. Frequently, constitutive activation of signalling pathways in those cells results in the induction of resistance to apoptosis. To target and reverse anti-apoptotic mechanisms is an attractive example of molecularly-targeted therapy. For example, members of the bcl-2 gene family and the "inhibitor of apoptosis protein"-families have been successfully targeted to render tumor cells more susceptible to apoptosis.

However, despite recent progress, there is an ongoing need to provide new and improved cancer therapies that can exploit both innate and adaptive immune responses.

### SUMMARY OF THE INVENTION

Provided herein are compositions and methods for targeting the evolutionarily conserved MDA-5-IPS-1 antiviral pathway in tumors to provoke parallel tumoricidal and immunostimulatory effects that bridge innate and adaptive immune responses for the therapeutic treatment of cancer.

An aspect of the present invention provides a MDA-5-encoding polynucleotide or a MDA-5 polypeptide, or a functional-conservative derivative thereof in a therapeutically effective amount for use in a method of treating and preventing recurrence of cancer in a subject in need thereof. In some embodiments, the cancer is prostate cancer. In particular embodiments, the MDA-5 includes one N-terminal caspase-recruitment domain (CARD) domain. In other embodiments, the MDA-5 does not include a CARD domain.

In some embodiments, the step of administering is performed by directly contacting tumor cells of said cancer with said MDA-5-encoding polynucleotide or a MDA-5 polypeptide, or a functional-conservative derivative thereof. In some embodiments, the method further comprises a step of simultaneously or sequentially administering one or more active agents selected from the group consisting of a MDA-7/IL-24-encoding polynucleotide, a MDA-7/IL-24 polypeptide, glucose-regulated protein 170 (grp170), grp170-flagellin hybrid chaperone (Flagrp170), and an immune checkpoint inhibitor. In some embodiments, the method further comprises a step of simultaneously or sequentially administering one or more chemotherapeutic or radiotherapeutic agents. In some embodiments, the MDA-5-encoding polynucleotide is regulated by a cancer selective promoter.

Disclosed is a method of inducing an immune-mediated response in a subject in need thereof, comprising administering to the subject a MDA-5-encoding polynucleotide or a MDA-5 polypeptide, or a functional-conservative derivative thereof in an amount sufficient to activate tumor-reactive cytotoxic T lymphocytes and/or natural killer cells in said subject.

Disclosed is a method of preventing recurrence of a cancer in a subject in remission, comprising administering to the subject a therapeutically effective amount of a Melanoma Differentiation-Associated gene 5 (MDA-5)-encoding polynucleotide or a MDA-5 polypeptide, or a functional-conservative derivative thereof.

### BRIEF DESCRIPTION OF THE DRAWINGS

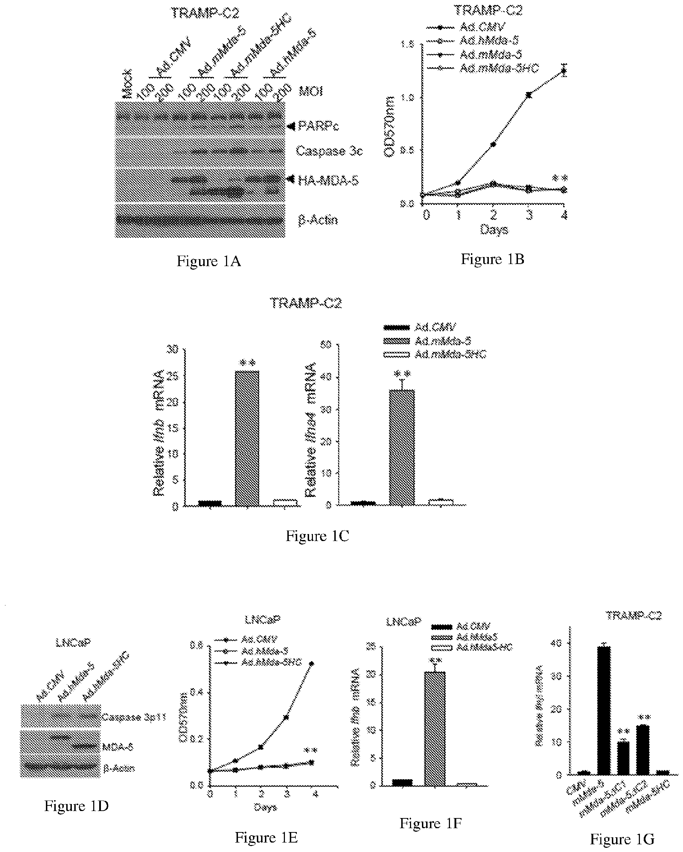

**Figure 1A-G****. Ectopic expression of MDA-5 results in cancer cell death and concomitant type I IFN production. A.** Adenoviral infection of mouse TRAMP-C2 tumor cells to express full length mouse MDA-5 (mMDA-5), human MDA5 (hMDA-5), or the CARDs-truncated mMDA-5 (mMDA-5HC) stimulated intrinsic cell death pathway, indicated by cleaved PARP (PARPc) and Caspase 3 (Caspase 3c, Asp175). **B.** Overexpression of MDA-5 or mMDA-5HC suppressed proliferation of TRAMP-C2 cells, analyzed by MTT assays. **C.** Deletion of CARD domains reduces the capacity of MDA-5 to induce IFN-β production. TRAMP-C2 cells were infected with indicated adenoviruses at a MOI of 200 for 90 min, followed by qRT-PCR analyses of the mRNA levels of IFN-β or IFN-α4 (^{∗∗}, *p*<0.01 *vs* CMV). **D-F.** Overexpression of full-length or CARD-truncated human MDA-5 in LNCaP cells activates Caspase 3 **(D)** and inhibits proliferation **(E).** Upregulation of the *Ifnb* gene was only observed in Ad*.hMDA-5* infected cells **(F). G.** CARDs domains are essential for MDA-5-initiated type I IFN production. Deletion of either the first CARD (i.e., mMDA-5ΔC1) or the second CARD (i.e., mMDA-5ΔC2) impaired MDA-5-induced elevation of the *Ifnb* gene in TRAMP-C2 cells. ^{∗∗}, *p*<0.01, *vs* mMDA-5. The experiments were repeated three times with similar results.

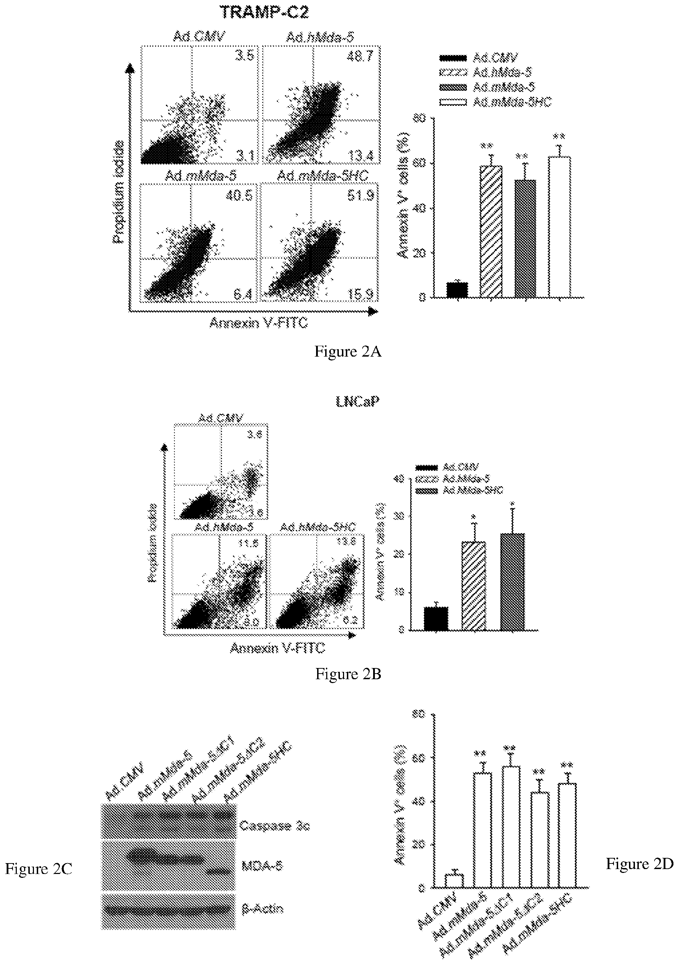

**Figure 2A-D****. Ectopic expression of full length MDA-5 or CARD domain truncated MDA-5 (MDA-5HC) induces apoptosis in cancer cells.** Mouse prostate cancer TRAMP-C2 cells **(A)** or human prostate cancer LNCaP cells **(B)** were infected with indicated adenoviruses at a MOI of 200 for 90 min. 48 h later, cell apoptosis was assessed using Annexin V/Propidium iodide staining and flow cytometry analyses. **(C-D)** CARD domains are dispensable for MDA-5-mediated tumoricidal effect. TRAMP-C2 cells were infected with indicated viruses encoding truncated MDA-5. Caspase 3 cleavage **(C)** and Annexin V positive cells **(D)** were assessed were measured by immunoblotting and flow cytometry, respectively. ^{∗}*, p*<0.05; ^{∗∗}*, p*<0.01.

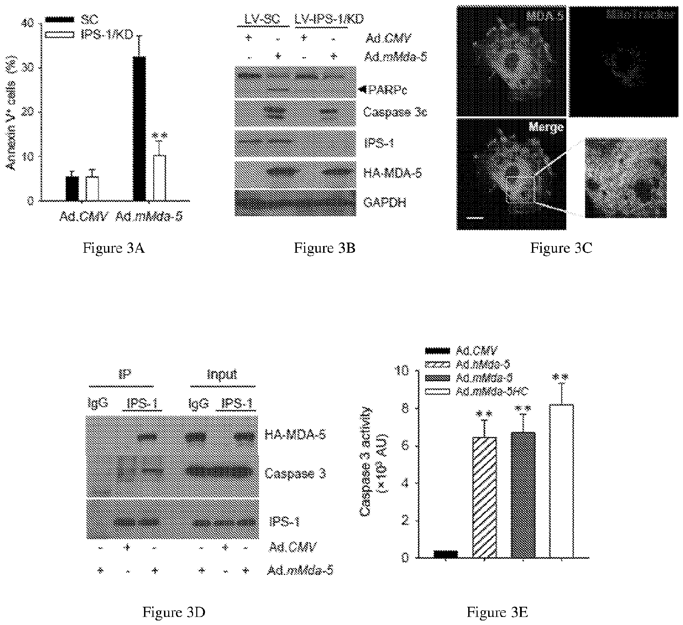

**Figure 3A-E****. IPS-1 is required for MDA-5-induced apoptosis in cancer cells. A-B.** Lentiviral shRNA-mediated knockdown (KD) of IPS-1 rendered TRAMP-C2 cells resistant to MDA5-induced apoptosis, assayed by Annexin V staining **(A)** and immunoblotting of cleaved PARP or Caspase 3 **(B).** Cells infected with lentiviral-encoded scrambled shRNA (LV-SC) were used as controls. C. Presence of expressed HA-tagged MDA-5 in mitochondria, as shown by confocal microscopy analysis of its co-localization with MitoTracker. Scale bar, 10 µm. **D.** MDA-5 directly associates with the adapter protein IPS-1 and promotes the IPS-1 interaction with Caspase 3. TRAMP-C2 cells were infected with Ad*.mMda-5* or Ad*.CMV* Cell lysates were immunoprecipitated with anti-IPS-1 antibodies, followed by immunoblotting with anti-HA antibodies (for HA-tagged MDA-5) or anti-Caspase 3 antibodies. **E.** Expression of MDA-5 or mMDA-5HC activates Caspase 3, with its enzymatic activity measured in cell extracts 48 h after infection. AU, arbitrary units. The experiments were repeated three times with similar results (^{∗∗}, *p*<0.01).

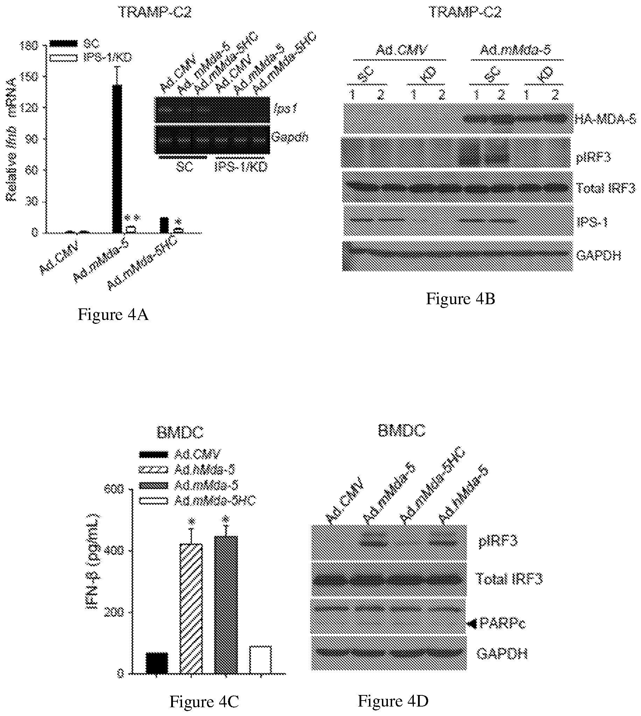

**Figure 4A-F****. MDA-5 induces IFN-β production in cancer cells and normal cells** *via* **IPS-1-IRF3 signaling pathway. A-B.** Stable knockdown of IPS-1 (KD) in TRAMP-C2 tumor cells abolished MDA-5-induced IFN-β expression **(A)** and IRF3 phosphorylation **(B). C-D.** MDA-5 expression-triggered type I IFN response is intact in normal cells. Primary BMDCs were subjected to ELISA assays for IFN-β production **(C)** or immunoblotting analyses for phosphorylation of IRF3 and cleaved PARP **(D)** after infection with indicated viruses. **E-F.** IPS-1-dependent IFN-β expression upon MDA-5 expression in normal cells. IPS-1-knockdown BMDCs **(E)** or IPS-1^{-/-} BMDCs **(F)** were infected with Ad*.CMV* or Ad*.mMda5,* followed by qRT-PCR analysis of IFN-β expression. LV-scrambled BMDCs (SC) or wild-type (WT) BMDCs were used as controls, respectively. The results are presented as fold over Ad.*CMV*-infected control cells. Data are means ± S.D. (*n* =3) and representative of three experiments (^{∗}, *p*<0.05; ^{∗∗}, *p*<0.01).

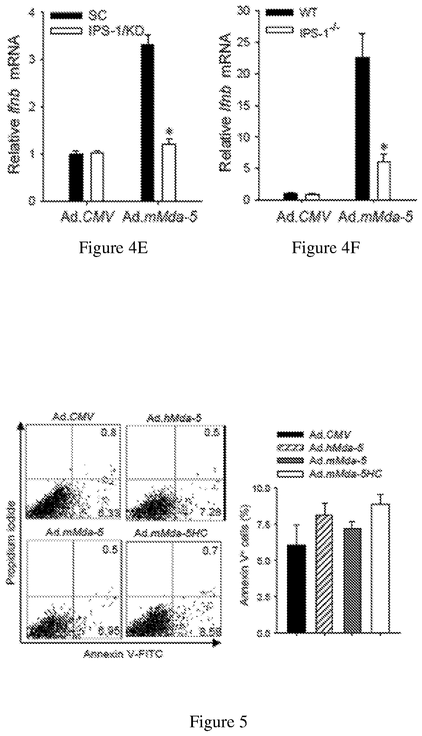

**Figure 5****. Ectopic expression of full length MDA-5 or CARD domain-truncated MDA-5 (MDA- 5HC) has little cytotoxic effect on normal cells.** Mouse bone marrow-derived dendritic cells (BMDCs) were infected with indicated adenoviruses at a MOI of 300 for 90 min. 48 h later, cell apoptosis was assessed using Annexin V/Propidium iodide staining and flow cytometry analyses.

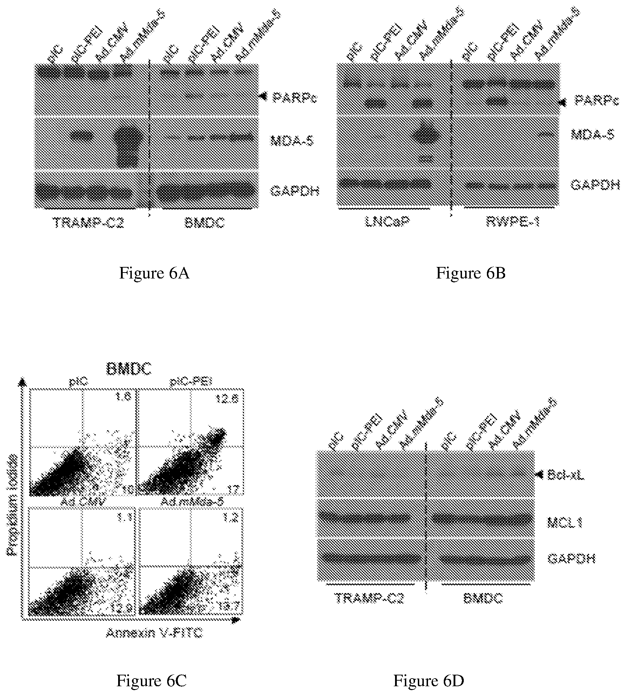

**Figure 6A-D****. Comparison of pIC-PEI and Ad.*Mda*-*5*-induced apoptosis in cancer and normal cells.** Mouse prostate cancer TRAMP-C2 cells and primary BMDCs **(A),** human prostate cancer LNCaP cells and normal human prostate epithelial cells RWPE-1 **(B)** were treated with naked poly(I:C) (pIC, 1 µg/mL), transfected with jetPEI-poly(I:C) (pIC-PEI, 1 µg/mL), or infected with indicated adenoviruses. PARP cleavage and MDA-5 expression levels were examined using immunoblotting. **C.** pIC transfection induced apoptosis in BMDCs, compared to infection with Ad*.mMda5,* indicated by Annexin V/PI double staining. **D.** TRAMP-C2 cells or primary BMDCs were treated with naked pIC, transfected with pIC-PEI, or infected with indicated adenoviruses. Bcl-xL and MCL1 levels were examined using immunoblotting.

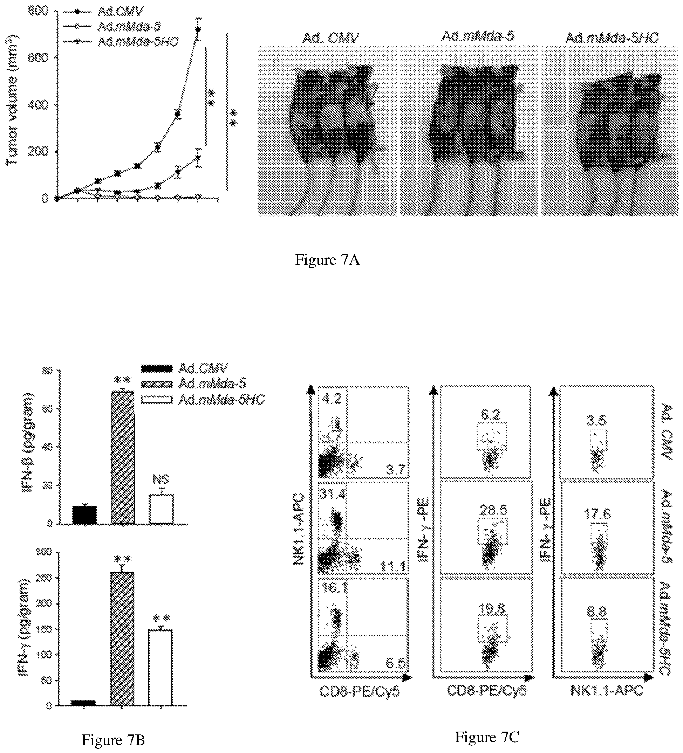

**Figure 7A-C****. *In situ* MDA-5 therapy eradicates mouse prostate cancer and potentiates innate and adaptive immune activation in tumor sites. A.** C57BL/6 mice established with C2-OVA tumors (n=5) were treated intratumorally with adenoviruses for a total of five doses. Ad*.mMda-5* therapy resulted in regression of tumors compared to those receiving Ad*.CMV* or Ad*.mMda-5HC.* Photographs of treated tumors at the end of the study are also shown. **B.** Elevation of IFN-β and IFN-γ levels in C2-OVA tumors following Ad*.mMda-5* therapy, as determined by tissue ELISA assays. C. MDA-5 therapy induces increased tumor infiltration by CD8⁺ T cells and NK1.1⁺ cells as well as enhanced IFN-γ production in these infiltrating cells, assayed by intracellular cytokine staining and FACS analyses. Data are representative of three independent experiments (^{∗∗}, *p*<0.01; NS, not significant).

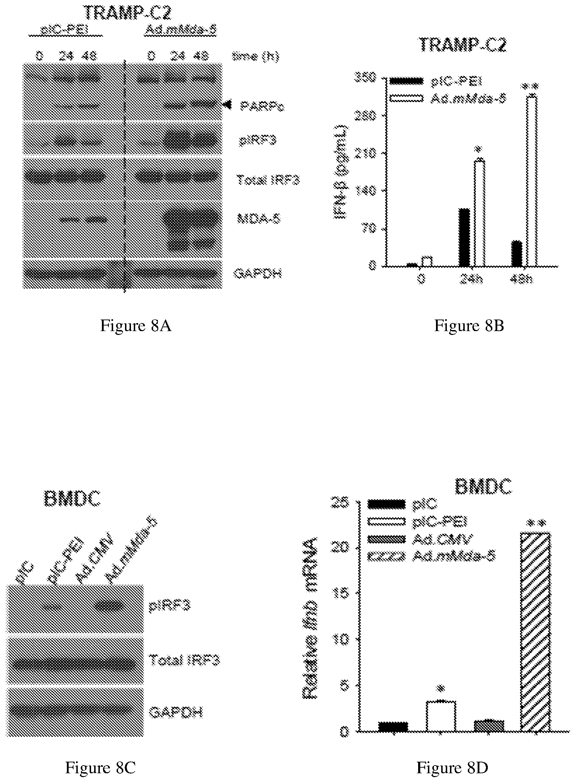

**Figure 8A-D****. Comparison of pIC-PEI and Ad.*Mda*-*5*-induced type I IFN signaling in cancer and normal cells. A.** Ad*.mMda-5* infection induced higher level of PARP cleavage and IRF3 phosphorylation in TRAMP-C2 cells as compared with **pIC-PEI** transfection. 24 or 48 h after **pIC-PEI** treatment or Ad*.mMda-5* infection, phosphorylation of IRF3 and cleavage of PARP were determined by immunoblotting. **B.** Ad*.mMda-5* infection induced higher and more sustained production of IFN-β in TRAMP-C2 cells than **pIC-PEI** transfection, assessed using ELISA. In addition, Ad*.mMda-5* infection induced higher level of IRF3 activation (C) and IFN-β production in BMDCs **(D),** as shown by immunoblotting and ELISA, respectively. Data are representative of two independent experiments. ^{∗}, *p*<0.05; ^{∗∗}, *p*<0.01

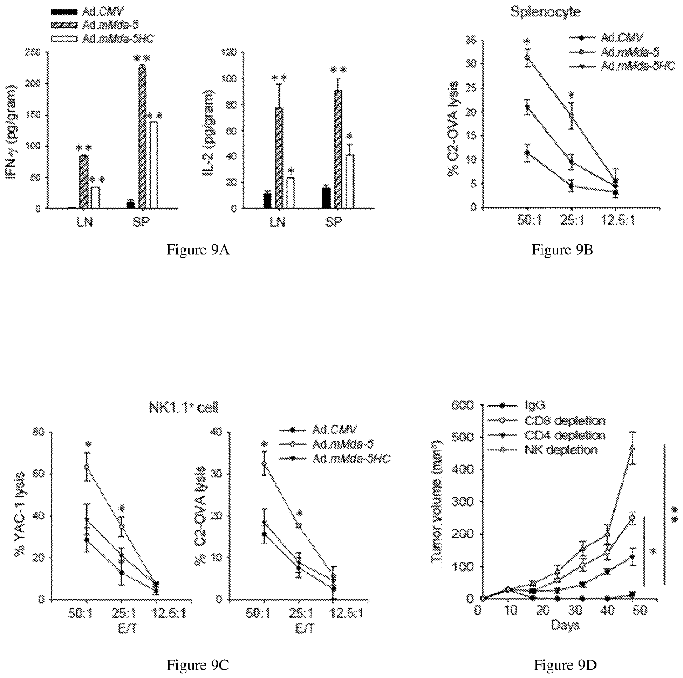

**Figure 9A-D****. *In situ* MDA-5 therapy generates a potent tumor-reactive CTL and NK cell response. A.** Lymph node (LN) cells or splenocytes (SP) from treated C2-OVA tumor bearing mice were stimulated with tumor cell lysates, followed by ELISA analyses of IFN-γ or IL-2 levels in the media. **B.** Increased cytotoxicity of effector T cells from Ad*.mMda-5-*treated mice. Pooled splenocytes (n=3) were cocultured with inactivated C2-OVA cells for 6 days in the presence of IL-2 (40IU/mL). The cytolytic activity of stimulated T cells was determined using a LDH release assay with live C2-OVA cells as targets. **C.** Cytotoxicity of NK cells was analyzed by mixing NK1.1⁺ cells sorted from the spleens (n=3) as effectors with C2-OVA or YAC-1 cells (1×10⁴) as targets at varying effector to target (E:T) ratios. Data shown are the mean percentage of killing from triplicates ± SD and are representative of two independent experiments. **D.** CD8⁺ cells and NK1.1⁺ cells are involved in the therapeutic activity of Ad*.mMda-5.* C2-OVA tumor-baring mice (n=5) were depleted of effector cell subsets with antibodies prior to MDA-5 therapy (^{∗}, *p*<0.05; ^{∗∗}, *p*<0.01).

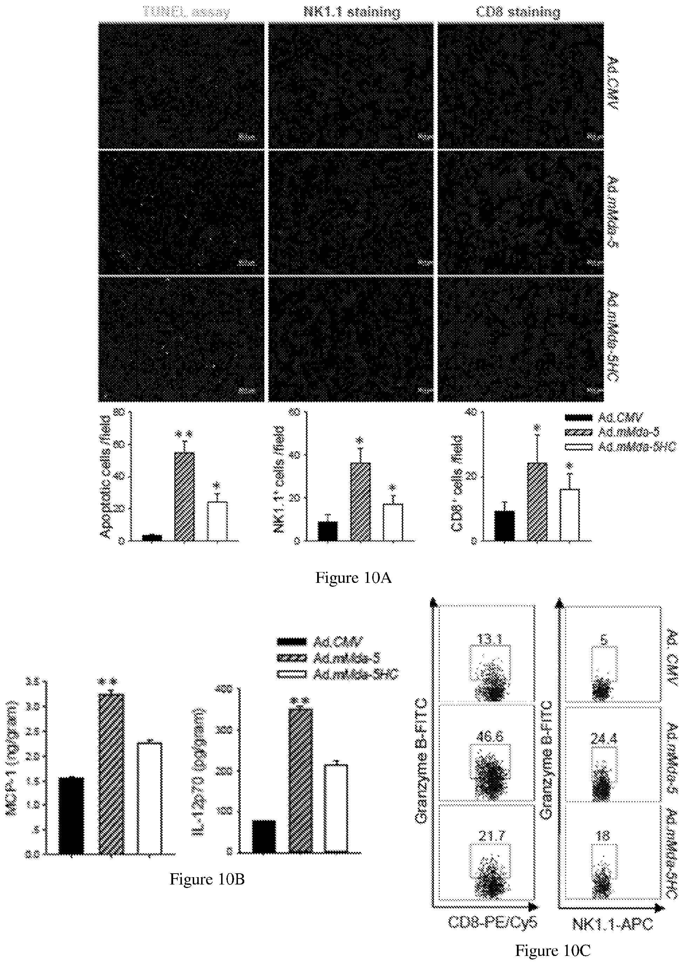

**Figure 10A-C****. *In situ* MDA-5 therapy promotes cancer cell death and immune activation in the tumor sites. A.** TRAMP-C2 tumor-bearing mice (n=5) were treated *i. t.* with Ads indicated for a total of five doses. TUNEL assays and immunofluorescence staining of tumor sections for CD8+ and NK1.1⁺ cells shows increased tumor cell death correlates with enhanced immune infiltration. The number of positive cells was quantified from ten randomized visual fields. Scale bars, 50 µm. **B.** Upregulation of MCP-1 (left) and IL-12 (right) expression in C2-OVA tumors following Ad*.mMda-5* therapy compared to treatment with either *Ad.CMV* or Ad*.mMda-5HC,* as determined by ELISA assays. C. Increased Granzyme B expression in tumor-infiltrating CD8+ T cells or NK cells, assayed using intracellular cytokine staining and FACS analysis. Data shown are representative of three independent experiments (^{∗}, *p*<0.05; ^{∗∗}, *p*<0.01).

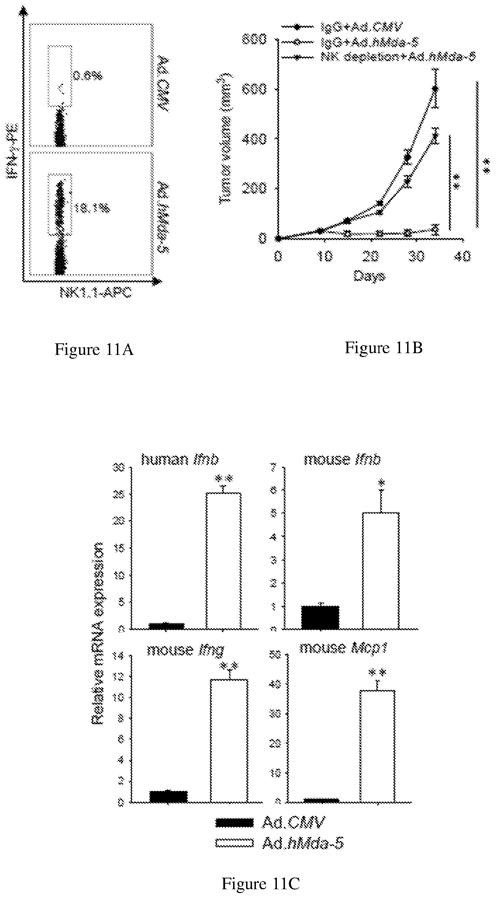

**Figure 11A-C****. *In situ* MDA-5 therapy suppresses human prostate cancer *via* NK cell activation. A.** Activation of NK cells by Ad*.hMda-5* therapy, indicated by intracellular IFN-γ staining of CD3⁻NK1.1⁺ cells isolated from LNCaP tumors in athymic nude mice. **B.** Depletion of NK1.1⁺ cells abolished the therapeutic activity of hMDA-5. Starting one day before treatment, mice (n=5) were given PK136 (200 µg/ml) or control IgG at weekly intervals. **C.** Elevation of human *Ifnb,* mouse *Ifnb, Ifng,* and *Mcp1* gene expression in LNCaP tumors after Ad*.hMda-5* therapy. Data shown are representative of two independent experiments (^{∗}*, p*<0.05*;* ^{∗∗}, *p*<0.01).

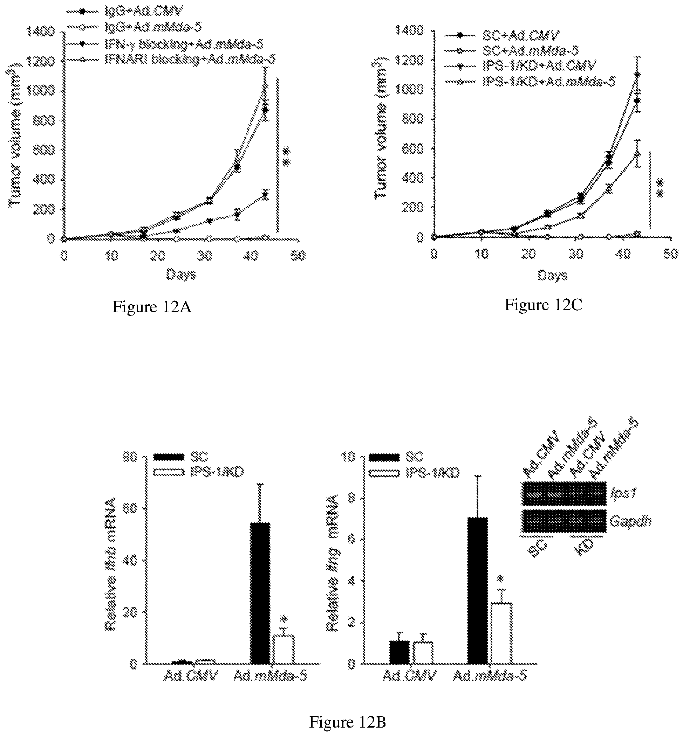

**Figure 12A-D****. Type I IFN pathway is crucial for *in situ* MDA-5 therapy-induced protective antitumor immunity. A.** Type I IFN signaling and IFN-γ are critical for tumor elimination by Ad*.mMda-5.* Antibody blockade of the IFN-α/β receptor (IFNAR-1) or neutralization of IFN-γ dampened the antitumor activity of Ad*.mMda-5* in C2-OVA tumor-bearing mice (n=5). **B.** Downregulation of IPS-1 expression in tumors by administration of a lentivirus encoding shRNA for gene silencing attenuated MDA-5-induced immune activation, indicated by reduced levels of IFN-β or IFN-γ in tumors. C. Silencing of IPS-1 abrogated the antitumor potency of Ad*.mMda-5.* Data shown are representative of two independent experiments (^{∗}, *p*<0.05; ^{∗∗}, *p*<0.01). **D.** Targeting the MDA-5-IPS-1 pathway in tumors to potentiate protective antitumor immunity. Overexpression of MDA-5, a sensor for viral dsRNA, in cancer cells triggers activation of IRF-3 and Caspase 3 in an IPS-1 dependent manner. The interaction between MDA-5 and its downstream adapter protein IPS-1 results in production of type I IFNs and concomitant cancer cell death. The type I IFNs promote the activation and effector functions of natural killer (NK) cells and/or cytotoxic T lymphocytes (CTLs). Collaborative action of tumor-reactive innate and adaptive immune cells leads to effective tumor eradication.

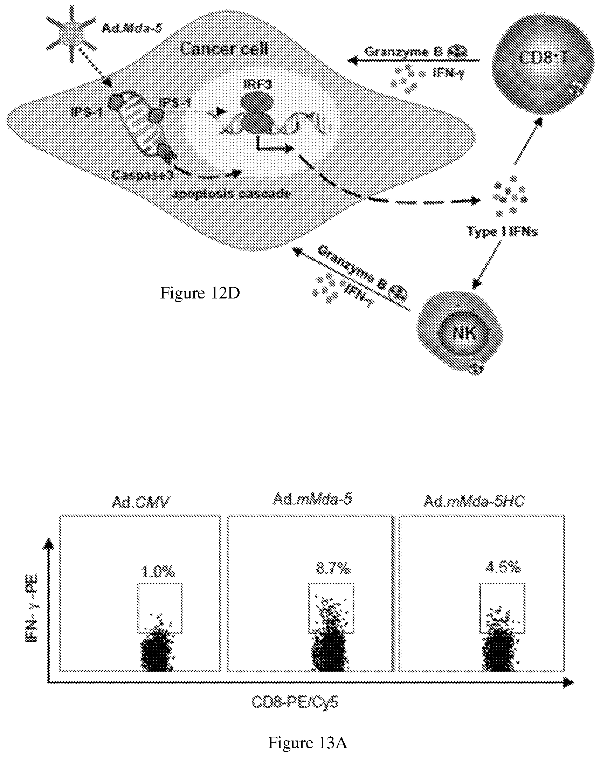

**Figure 13A-C****. *In situ* MDA-5 therapy generates systemic antigen-specific CTL response and long-term immune memory. A.** After treatment with indicated viruses, splenocytes from C2-OVA tumor-bearing mice were stimulated with MHC I-restricted OVA254-267 peptide (1 µg/mL) for 3 days and subjected to intracellular cytokine staining for determining the frequency of IFN-γ-producing CD8+ CTLs. **B.** C2-OVA tumor-free mice that have rejected the secondary tumor challenge showed a potent T cell response, as determined by qRT-PCR analysis of IFN-γ expression in the spleen **(B)** or by ELISA assays for OVA peptide-induced IFN-γ production **(C).** Data are representative of two independent experiments. ^{∗}, *p*<0.05; ^{∗∗}, *p*<0.01.

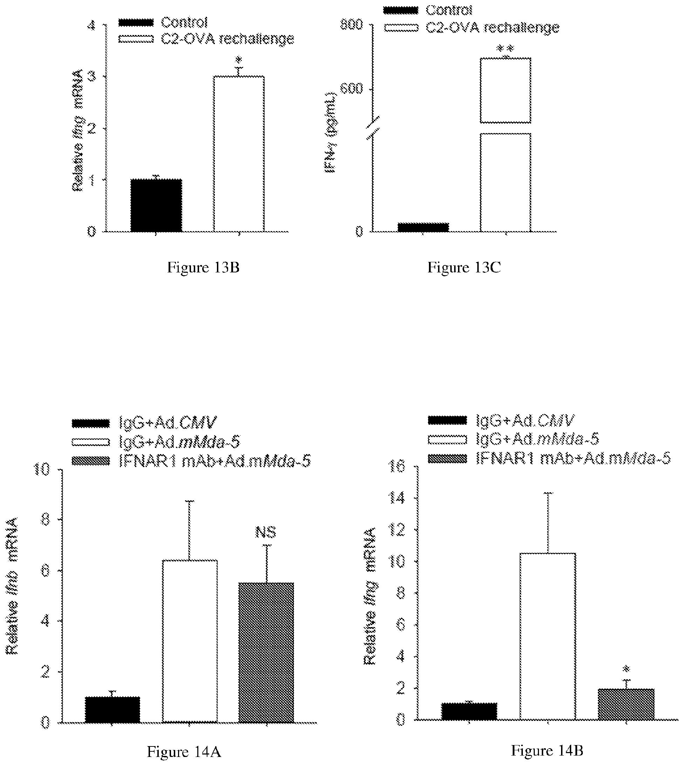

**Figure 14A-B****.** Antibody blockade of IFNAR-1 in TRAMP-C2 tumorbearing mice does not affect *in situ* Ad*.mMda-5* therapy-induced IFN-β production **(A),** but markedly reduces the levels of IFN-γ in the tumor site **(B).** Data are representative of two independent experiments. ^{∗}, *p*<0.05. NS, not significant

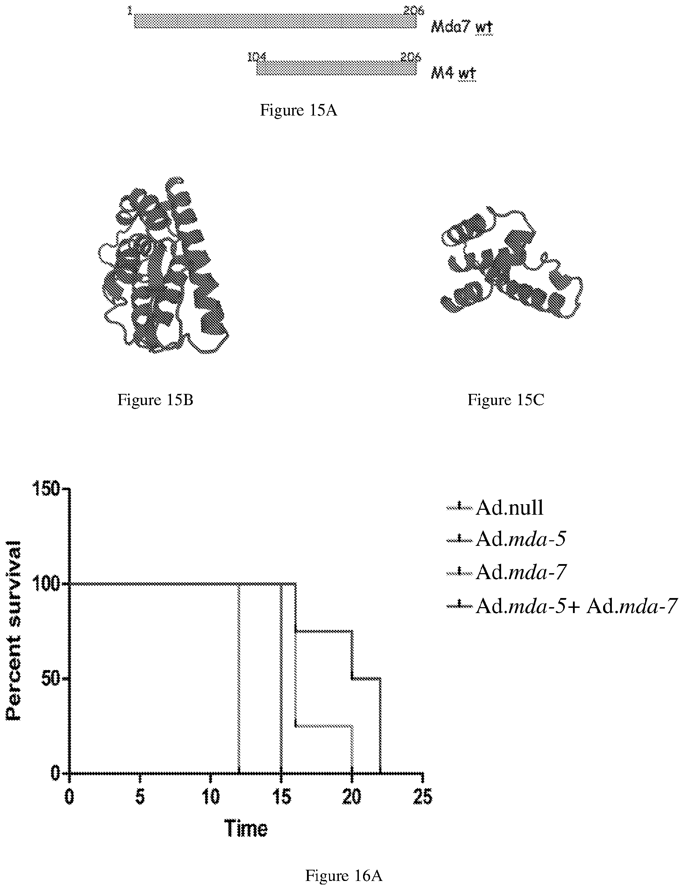

**Figure 15A-C****. (A)** Schematic diagram of MDA-7 and M4. Structure prediction model using SWISS-MODEL **of (B)** MDA-7 and **(C)** M4.
**Figure 16A-C****.** Combined effect of mda-5 and mda-7/IL-24 on **(A)** survival, **(B)** tumor size, and **(C)** tumor weight in B16 mouse melanoma xenograft studies.

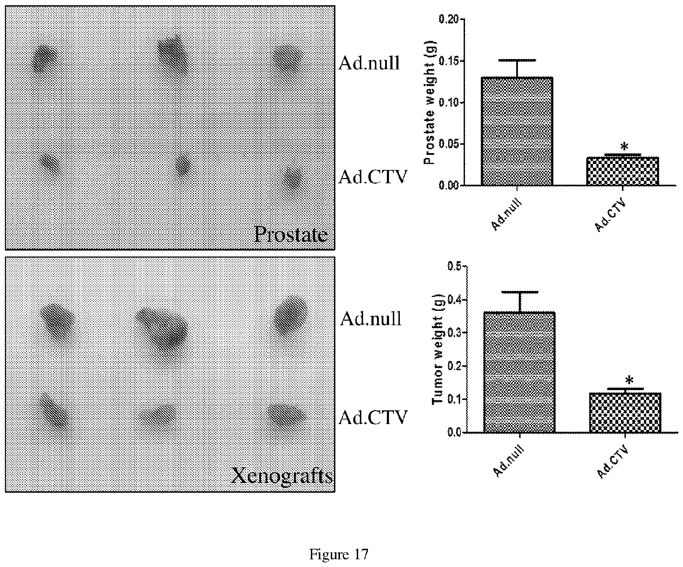

**Figure 17****.** Transgenic mouse model for prostate cancer. Hi-MYC mice were implanted with Hi-MYC cells subcutaneously (0.5^{∗}10⁵) and treated with Ad.CTV (10⁸vp for 8 doses). The mice were sacrificed and prostate was isolated. The tumor (prostate/ xenografts) weights were measured and graph was plotted.

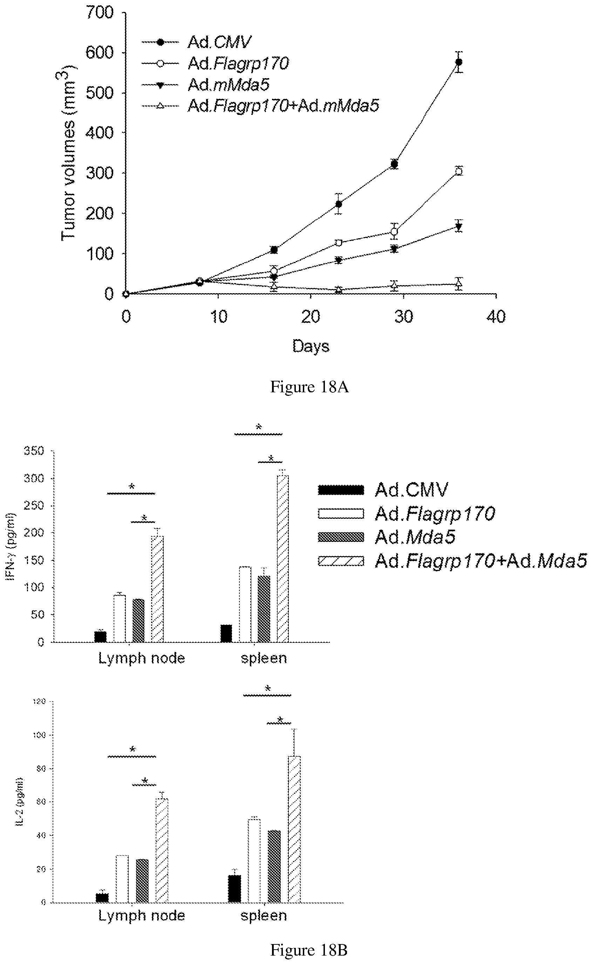

**Figure 18A-B****.** Combination therapy with Ad. *MDA5* plus Ad. *Flagrp170* generate a potent antitumor CTL immune response. **(A)** Mice established with prostate tumor TRAMP-C2-OVA (n=5) were treated with the indicated adenoviruses every 3 days for a total 5 doses. Tumor growth was monitored by measuring tumor sizes. **(B)** Lymphocytes from treated mice were stimulated with 1 µg/ml OVA peptide. IFN-γ and IL-2 levels in the culture media were assessed using ELISA. ^{∗}, *p*<0.05

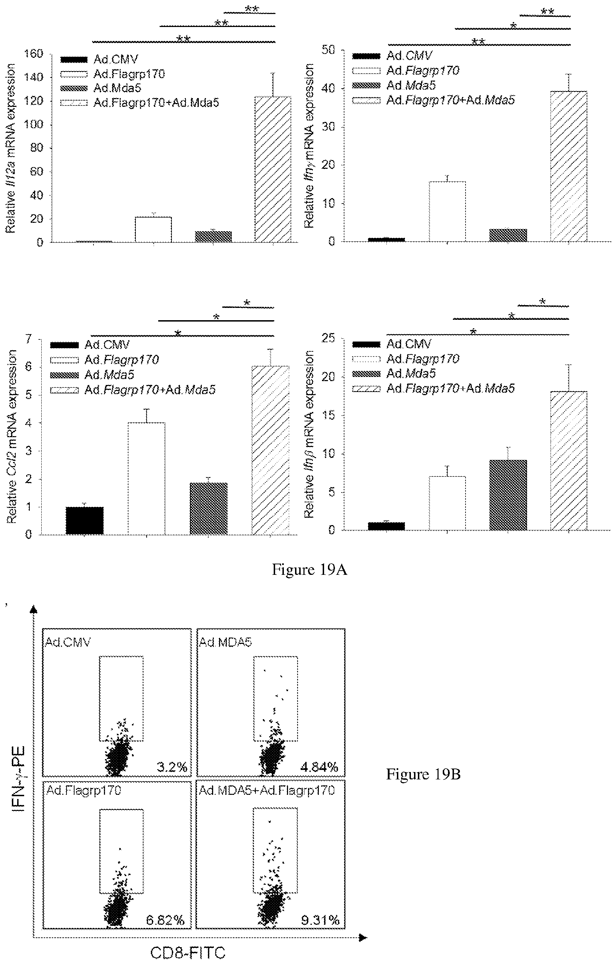

**Figure 19A-B****.** Combination therapy promotes immune activation in the tumor sites. Mice established with prostate tumor TRAMP-C2-OVA (n=5) were treated with the indicated adenoviruses for a total 5 doses. **(A)** Elevation of intratumoral IL-12p35, IFN-γ, CCL-2, and IFN-β was assessed using qRT-PCR. (^{∗}, *p*<0.05; ^{∗∗}, *p*<0.01) **(B)** Three days after last treatment, lymphocytes from treated mice were stimulated with OVA peptide. The frequency of IFN-γ-producing, tumor-infiltrating CD8⁺ T cells was examined with intracellular cytokine staining and FACS.

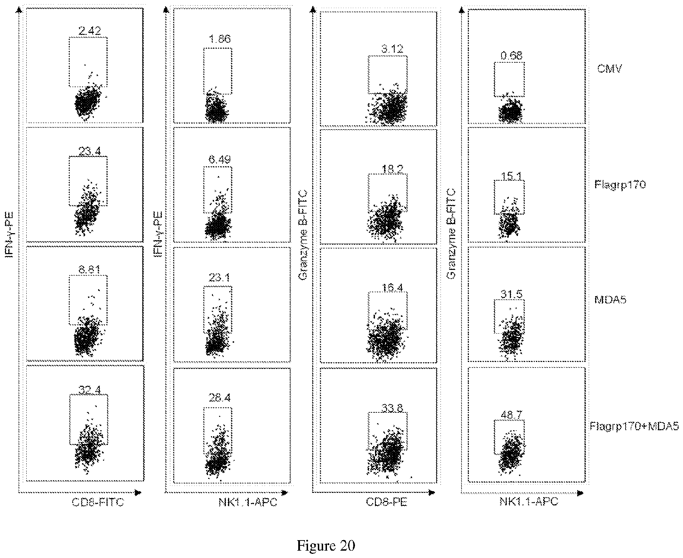

**Figure 20****.** Programing of the tumor microenviroment by combination therapy with Ad.MDA-5 plus Ad.Flagrp170. The Th1 polarizing effect of combinatorial therapy on the tumor environment is indicated by profoundly increased tumor infiltration by IFN-γ and granzyme B-producing CD8⁺ CTLs and NK cells.

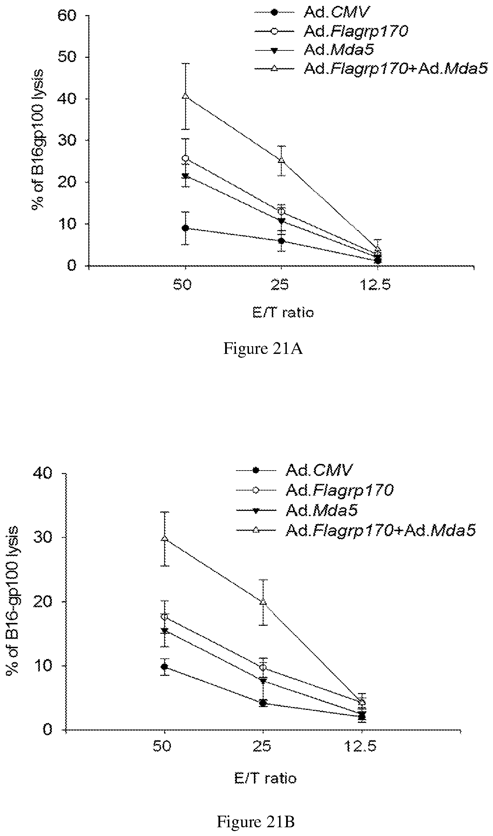

**Figure 21A-B****.** Combination therapy with Ad. MDA5 plus Ad.Flagrp170 generate the most potent antitumor effector CTLs and NK cells. **(A).** Splenocytes pooled from two mice of each group were stimulated with mitomycin C-treated B16 cells at a ratio of 2:1 and IL-2 (40 IU/ml) in complete culture medium for 5 days. Viable cells were isolated by percoll density gradient centrifugation and then incubated with B16 tumor cells at 37°C for 6 h. Cytotoxicity of splenic CTLs was measured using CytoTox 96 Non-Radioactive Cytotoxicity Assay Kit. **(B).** NK cells were sorted from the spleen of tumor bearing mice after treatment. The cytotoxicity of NK cells on B16 target cells was determined using the same kit.

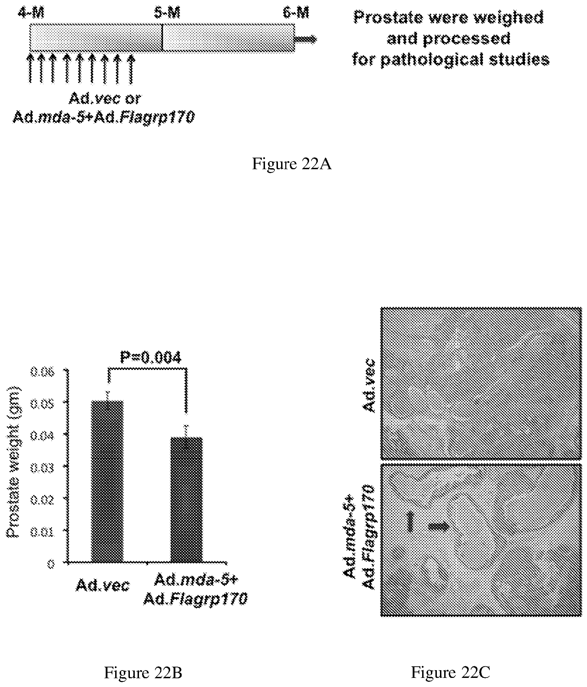

**Figure 22A-C****.** Ad*.mda-5* in combination with Ad.*Flagrp170* significantly reduces prostate tumor development in *Hi-myc* transgenic mice. **A.** Schematic diagram of experimental protocol was presented. Mice received either Ad*.vec* or therapeutic viruses complexed with decorated microbubble through intravenous injections. For sonoporation, MicroMaxx^{®} Ultrasound System with L25-e probe (SonoSite, Inc. Bothell, WA, USA) was used. The experiment was terminated after two months and mice were sacrificed and the prostates were collected. **B.** Tumor containing prostates were weighed (shown in grams). Graph depicts average tumor weights of the two groups. Two tail T-test was done and p value was determined. **C.** H&E staining of representative prostate sections of Hi*-myc* mice at lower magnification are presented. In lower panel photographs normal-like prostate glands are indicated (arrow).

### DETAILED DESCRIPTION OF THE INVENTION

Innate pattern recognition receptors (PRRs), such as RIG-I-like receptor and toll-like receptors, are specialized for recognizing pathogen-associated molecular patterns and play essential roles in host immunity (1). Melanoma differentiation associated gene-5 (*mda-5*) or Helicard, initially identified as a type I interferon (IFN)-inducible gene in human melanoma by subtraction hybridization (2), is a cytoplasmic RIG-I-like receptor and now considered as a first line of defense against viral infection by sensing viral double-stranded RNA (dsRNA) (3). The MDA-5 protein consists of two N-terminal tandem caspase-recruitment domains (CARDs), the central DExD/H-box motif helicase domain, and C-terminal domain. Upon recognition of viral dsRNA, MDA-5 binds to the adapter protein IFN-β promoter stimulator 1 (IPS-1), which is tethered to the outer mitochondrial membrane (4). This interaction *via* CARD domains triggers a complex signaling cascade involving transcription factors, NF-κB and IFN regulatory factor 3 (IRF-3) in particular, resulting in expression of the type I IFNs and IFN-stimulated genes to initiate antiviral immune responses (5-8). MDA-5 can also execute its antiviral activity by inducing apoptosis of virus-infected cells in a type I IFN-independent manner (9, 10). In this context, cleavage of the MDA-5 protein and subsequent nuclear translocation of the helicase domain further accelerates DNA fragmentation (11).

The aspects of the invention are defined in the claims.

As described in Example 1, the biological effects of MDA-5, *via* ectopic expression of human or mouse MDA-5 protein, on selective induction of prostate cancer cell death and activation of type I IFN response have been investigated. The Example provides new insight into the structural domains of MDA-5 that confer direct tumoricidal or type I IFN-promoting effect upon its expression in cancer cells. In particular, it was found that MDA-5 without either of the two N-terminal CARD domains or with only one of the CARD domains is sufficient for its pro-apoptotic effects. However, MDA-5 or variants containing either one of the two CARD domains was required to stimulate IFN signaling.

Moreover, the present invention demonstrates for the first time the superior antitumor efficacy of *in situ* MDA-5 therapy in eradicating established prostate cancers. Without being bound by theory, the mechanistic studies reveal that this enhanced tumor control is mediated primarily by engaging type I IFN response in the tumor site *via* MDA-5-IPS-1 axis, which results in systemic mobilization of both innate and adaptive components of the immune system.

The human *mda-5* DNA (SEQ ID NO:1) and amino acid (SEQ ID NO:2) sequences are found at GenBank # NM_022168. The mouse *mda-5* DNA (SEQ ID NO:3) and amino acid (SEQ ID NO:4) sequences are found at GenBank accession # NM_027835.

A "coding sequence" or a sequence "encoding" an expression product, such as a RNA, polypeptide, protein, or enzyme, is a nucleotide sequence that, when expressed, results in the production of that RNA, polypeptide, protein, or enzyme, *i.e.,* the nucleotide sequence encodes an amino acid sequence for that polypeptide, protein or enzyme.

By "purified" and "isolated" it is meant, when referring to a polypeptide or a polynucleotide sequence, that the indicated molecule is present in the substantial absence of other biological macromolecules of the same type. The term "purified" as used herein preferably means at least 75% by weight, more preferably at least 85% by weight, still preferably at least 95% by weight, and most preferably at least 98% by weight, of biological macromolecules of the same type are present. An "isolated" nucleic acid molecule which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

The terms "subject" and "patient" are used interchangeably herein, and refer to an animal such as a mammal, which is afflicted with or suspected of having, at risk of, or being pre-disposed to cancer. The terms may refer to a human. The terms also include domestic animals bred for food, sport, or as pets, including horses, cows, sheep, poultry, fish, pigs, cats, dogs, and zoo animals, goats, apes (e.g. gorilla or chimpanzee), and rodents such as rats and mice. Typical subjects include persons susceptible to, suffering from or that have suffered from cancer.

As used herein, the terms "cancer", "hyperproliferative" and "neoplastic" refer to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. The terms "cancer" or "neoplasms" include malignancies of the various organ systems, such as malignancies affecting lung, breast, skin, thyroid, lymphoid tissues, gastrointestinal, and genito-urinary tracts, that include, but are not limited to, adenocarcinomas of the colon, prostate, lung and/or testicular tumors, non-small cell carcinoma of the lung, cancer of the small intestine and of the esophagus.

The term "cancer metastasis" has its general meaning in the art and refers to the spread of a tumor from one organ or part to another non-adjacent organ or part.

The polypeptides may be produced by any technique known per se in the art, such as, without limitation, any chemical, biological, genetic or enzymatic technique, either alone or in combination. The disclosure thus provides an isolated, synthetic or recombinant MDA-5 polypeptide or a functional-conservative derivative thereof.

Knowing the amino acid sequence of the desired sequence, one skilled in the art can readily produce said polypeptides, by standard techniques for production of polypeptides. For instance, they can be synthesized using well-known solid phase methods, preferably using a commercially available peptide synthesis apparatus (such as that made by Applied Biosystems, Foster City, California) and following the manufacturer's instructions.

Alternatively, the polypeptides can be synthesized by recombinant DNA techniques as is now well-known in the art. For example, these fragments can be obtained as DNA expression products after incorporation of DNA sequences encoding the desired (poly)peptide into expression vectors and introduction of such vectors into suitable eukaryotic or prokaryotic hosts that will express the desired polypeptide, from which they can be later isolated using well-known techniques.

Polypeptides can be used in an isolated (e.g., purified) form or contained in a membrane or lipid vesicle (e.g. a liposome).

Functional-conservative derivatives or variants may result from modifications and changes that may be made in the structure of the polypeptides (and in the DNA sequences encoding it), and still obtain a functional molecule with desirable characteristics (e.g. tumoricidal and/or immunostimulatory effects).

Accordingly, functional-conservative derivatives or variants are those in which a given amino acid residue in a protein has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A functional-conservative derivative also includes a polypeptide which has at least 60 % amino acid identity as determined by BLAST or FASTA algorithms, preferably at least 75 %, more preferably at least 85%, still preferably at least 90 %, and even more preferably at least 95%, and which has the same or substantially similar properties or functions as the native or parent protein to which it is compared. Two amino acid sequences are "substantially homologous" or "substantially similar" when greater than 80 %, preferably greater than 85 %, preferably greater than 90 % of the amino acids are identical, or greater than about 90 %, preferably greater than 95 %, are similar (functionally identical). Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

The amino acid changes may be achieved by changing codons in the DNA sequence, according to Table 1.

**Table 1**

| **Amino acids** | | | **Codons** |
|---|---|---|---|
| Alanine | Ala | A | GCA, GCC, GCG, GCU |
| Cysteine | Cys | C | UGC, UGU |
| Aspartic Acid | Asp | D | GAC, GAU |
| Glutamic acid | Glu | E | GAA, GAG |
| Phenylalanine | Phe | F | UUC, UUU |
| Glycine | Gly | G | GGA, GGC, GGG, GGU |
| Histidine | His | H | CAC, CAU |
| Isoleucine | Ile | I | AUA, AUC, AUU |
| Lysine | Lys | K | AAA, AAG |
| Leucine | Leu | L | UUA, UUG, CUA, CUC, CUG, CUU |
| Methionine | Met | M | AUG |
| Asparagine | Asn | N | AAC, AAU |
| Proline | Pro | P | CCA, CCC, CCG, CCU |
| Glutamine | Gln | Q | CAA, CAG |
| Arginine | Arg | R | AGA, AGG, CGA, CGC, CGG, CGU |
| Serine | Ser | S | AGC, AGU, UCA, UCC, UCG, UCU |
| Threonine | Thr | T | ACA, ACC, ACG, ACU |
| Valine | Val | V | GUA, GUC, GUG, GUU |
| Tryptophan | Trp | W | UGG |
| Tyrosine | Tyr | Y | UAU |

For example, certain amino acids may be substituted for other amino acids in a protein structure without appreciable loss of tumoricidal effects. Since it is the interactive capacity and nature of a protein that defines that protein's biological functional activity, certain amino acid substitutions can be made in a protein sequence, and, of course, in its DNA encoding sequence, and nevertheless obtain a protein with like properties. It is thus contemplated that various changes may be made in the polypeptide sequences of the invention, or corresponding DNA sequences which encode said polypeptides, without appreciable loss of their biological activity. Said tumoricidal activity and immunostimuolatory activity can be assessed by various techniques well-known in the art, such as for instance the assays referred in the Example.

As outlined above, amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

In specific embodiments, it is contemplated that polypeptides for use according to the invention may be modified in order to improve their therapeutic efficacy. Such modification of therapeutic compounds may be used to decrease toxicity, increase circulatory time, or modify biodistribution. For example, the toxicity of potentially important therapeutic compounds can be decreased significantly by combination with a variety of drug carrier vehicles that modify biodistribution.

A strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain. For example, Pegylation is a well established and validated approach for the modification of a range of polypeptides. Therefore, the polypeptides may be covalently linked with one or more polyethylene glycol (PEG) group(s).

In another embodiment the polypeptides are covalently coupled to a tumor targeting agent as well known in the art. Non limiting examples include but are not limited to antibodies directed against the EDB domain of fibronectin, antibodies or agents binding Vascular endothelial growth factor receptor 2, antibodies or molecules binding fibroblast growth factor receptor-1, antibodies or agents that interact with CD31, antibodies or agents interacting with tumor lymphatic endothelium (Podoplanin, Lyve-1), or antibodies or agents binding to αVβ3 integrin such as RGD peptides, or antibodies or agents interacting with tumor membrane-bound and intracellular targets.

Another aspect is an isolated, synthetic or recombinant nucleic acid encoding for a MDA-5 polypeptide for use according to the invention. Typically, said nucleic acid is a DNA or RNA molecule, which may be included in any suitable vector, such as a plasmid, nanoparticle, cosmid, episome, artificial chromosome, phage or a viral vector. The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

Such vectors may comprise regulatory elements, such as a promoter, enhancer, terminator and the like, to cause or direct expression of said polypeptide upon administration to a subject. The vectors may further comprise one or several origins of replication and/or selectable markers. The promoter region may be homologous or heterologous with respect to the coding sequence, and provide for ubiquitous, constitutive, regulated and/or tissue specific expression, in any appropriate host cell, including for *in vivo* use. Examples of promoters include bacterial promoters (T7, pTAC, Trp promoter, etc.), viral promoters (LTR, TK, CMV-IE, etc.), mammalian gene promoters (albumin, PGK, etc), and the like.

By using cancer-selective promoters, MDA-5 can be targeted directly in cancers resulting in cancer cell death and induction of interferon-beta, resulting in stimulation of an cancer-specific immune response. Examples of cancer-selective promoters include progression elevated gene-3, astrocyte elevated gene-1, survivin, telomerase, truncated CCN1, melanoma differentiation associated gene-9/syntenin-1, etc.

Examples of plasmids include replicating plasmids comprising an origin of replication, or integrative plasmids, such as for instance pUC, pcDNA, pBR, and the like. Examples of viral vectors include adenoviral, retroviral, herpes virus, vaccinia virus and AAV vectors. Such recombinant viruses may be produced by techniques known in the art, such as by transfecting packaging cells or by transient transfection with helper plasmids or viruses.

There are several ways in which vectors can be administered or "directly contacted" with target cells. The term "directly contacting" can refer to intratumoral delivery of the therapeutic agent. Approaches to define cancer-selective expression include using cancer-selective promoters to regulate expression, use of nanoparticles containing PEI to facilitate cell uptake and delivery in liposomes, ultrasound-targeted microbubble-destruction (UTMD) approach, or using cellular delivery vehicles (dendritic cells and T-cells).

For *ex vivo* somatic gene therapy, the target cells are removed from the body, cultured in the laboratory with a vector, and re-inserted into the body. This process is usually carried out using blood cells since it is simple to remove and return. For *in situ* somatic gene therapy, the vector is placed directly into the affected tissue. For *in vivo* somatic gene therapy,
the vector is injected into the bloodstream , and is able to find and insert new genes only into the cells for which it was specifically designed. Vectors used in gene therapy can be classified as viral or non-viral.

For example, in one embodiment, the MDA-5 gene is administered to tumor sites for *in situ* therapy and induces systemic tumor-specific immune responses, resulting in efficient eradication of both treated primary tumors and distant cancer metastases.

In another embodiment, cancer cell lines or tumor cells derived from patients are genetically modified with MDA-5. MDA-5-expressing cancer cells are then irradiated and used as cell vaccines to immunize cancer patients for induction of cancer-specific immune responses.

In another embodiment, methods such as ultrasound-targeted microbubble-destruction (UTMD) allow for direct delivery of MDA-5 to tumors in a defined manner. By destroying targeted tumors, a systemic immune response resulting in eradication of metastasis will occur.

The term "treating" or "treatment", as used herein, means reversing, alleviating, inhibiting the progress of, or ameliorating the disorder or condition to which such term applies, or one or more symptoms of such disorder or condition.

The term "prevent" or "prevention" refers to any success or indicia of success in the forestalling or delay of cancer recurrence/relapse in patients in clinical remission, as measured by any objective or subjective parameter, including the results of a radiological or physical examination. The patient may have cancer at the time of treatment (and thus future recurrence of the cancer is prevented) or may be in remission, e.g. after treatment with the agents of the invention and/or another course of therapy. Thus, the agents may be used as an anti-cancer vaccine.

By a "therapeutically effective amount" is meant a sufficient amount of the molecule to treat a cancer, (for example, to limit tumor growth or to slow or block tumor metastasis) at a reasonable benefit/risk ratio applicable to any medical treatment. It will be understood, however, that the total daily usage of the molecules and compositions will be decided by the attending physician within the scope of sound medical judgment. The specific therapeutically effective dose level for any particular subject will depend upon a variety of factors including the disorder being treated and the severity of the disorder; activity of the specific polypeptide employed; the specific composition employed, the age, body weight, general health, sex and diet of the subject; the time of administration, route of administration, and rate of excretion of the specific polypeptide employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved.

In a particular embodiment the polypeptide or nucleic acid for use according to the invention may be administered sequentially or concomitantly with one or more active agents selected from the group consisting of a Melanoma Differentiation-Associated gene 7 (MDA-7)-encoding polynucleotide, a MDA-7 polypeptide, glucose-regulated protein 170 (grp170), grp170-flagellin hybrid chaperone (Flagrp170), or a functional-conservative derivative thereof, and an immune checkpoint inhibitor, such as a PD-1, PD-L1, B7-1/B7-2, or CTLA-4 inhibitor. An important part of the immune system is its ability to tell between normal cells in the body and those it sees as "foreign." This lets the immune system attack the foreign cells while leaving the normal cells alone. To do this, it uses "checkpoints" - molecules on certain immune cells that need to be activated (or inactivated) to start an immune response. Cancer cells can use these checkpoints to avoid being attacked by the immune system and thus drugs that target these checkpoints can be used as a cancer treatment. Exemplary PD-1 inhibitors include, but are not limited to, Pembrolizumab and Nivolumab. An exemplary PD-L1 inhibitor includes, but is not limited to, Atezolizumab. In some embodiments, the polypeptide or nucleic acid works synergistically with an active agent.

The DNA and amino acid sequences of MDA-7 are represented by SEQ ID NO: 6 and SEQ ID NO: 7, respectively. A functional-conservative derivative of MDA-7 includes, but it not limited to, a "therakine", which comprises a portion, or active component, of MDA-7with a Flt-3 secretory motif, that has been shown to display properties similar to MDA-7. The portion of MDA-7 present in the therakine is termed M4 with the DNA and amino acid sequences represented by SEQ ID NO: 8 and SEQ ID NO: 9, respectively. A schematic diagram and structure prediction models of MDA-7 and M4 are shown in Figure 15A-C.

In a particular embodiment the polypeptide or nucleic acid may be administered sequentially or concomitantly with one or more chemotherapeutic or radiotherapeutic agents.

In one embodiment said chemotherapeutic or radiotherapeutic agents are a therapeutic active agent used as anticancer agent. For example, said anticancer agents include but are not limited to fludarabine, gemcitabine, capecitabine, methotrexate, mercaptopurine, thioguanine, hydroxyurea, cytarabine, cyclophosphamide, ifosfamide, nitrosoureas, platinum complexes such as cisplatin, carboplatin and oxaliplatin, mitomycin, dacarbazine, procarbazine, epipodophyllotoxins such as etoposide and teniposide, camptothecins such as irinotecan and topotecan, bleomycin, doxorubicin, idarubicin, daunorubicin, dactinomycin, plicamycin, mitoxantrone, L-asparaginase, doxorubicin, epirubicin, 5-fluorouracil and 5-fluorouracil combined with leucovorin, taxanes such as docetaxel and paclitaxel, levamisole, estramustine, nitrogen mustards, nitrosoureas such as carmustine and lomustine, vinca alkaloids such as vinblastine, vincristine, vindesine and vinorelbine, imatinib mesylate, hexamethylmelamine, kinase inhibitors, phosphatase inhibitors, ATPase inhibitors, tyrphostins, protease inhibitors, inhibitors herbimycin A, genistein, erbstatin, and lavendustin A. In one embodiment, additional anticancer agents may be selected from, but are not limited to, one or a combination of the following class of agents: alkylating agents, plant alkaloids, DNA topoisomerase inhibitors, anti-folates, pyrimidine analogs, purine analogs, DNA antimetabolites, taxanes, podophyllotoxins, hormonal therapies, retinoids, photosensitizers or photodynamic therapies, angiogenesis inhibitors, antimitotic agents, isoprenylation inhibitors, cell cycle inhibitors, actinomycin, bleomycin, anthracyclines, MDR inhibitors and Ca²⁺ ATPase inhibitors.

Additional anticancer agents may be selected from, but are not limited to, cytokines, chemokines, growth factors, growth inhibitory factors, hormones, soluble receptors, decoy receptors, monoclonal or polyclonal antibodies, mono-specific, bi-specific or multi-specific antibodies, monobodies, polybodies.

Further therapeutic active agents may be an antiemetic agent. Suitable antiemetic agents include, but are not limited to, metoclopramide, domperidone, prochlorperazine, promethazine, chlorpromazine, trimethobenzamide, ondansetron, granisetron, hydroxyzine, acetylleucine, alizapride, azasetron, benzquinamide, bietanautine, bromopride, buclizine, clebopride, cyclizine, dimenhydrinate, diphenidol, dolasetron, meclizine, methallatal, metopimazine, nabilone, pipamazine, scopolamine, sulpiride, tetrahydrocannabinols, thiethylperazine, thioproperazine and tropisetron. In a preferred embodiment, the antiemetic agent is granisetron or ondansetron.

In still another embodiment, the other therapeutic active agent can be an opioid or non-opioid analgesic agent. Suitable opioid analgesic agents include, but are not limited to, morphine, heroin, hydromorphone, hydrocodone, oxymorphone, oxycodone, metopon, apomorphine, buprenorphine, meperidine, loperamide, ethoheptazine, betaprodine, diphenoxylate, fentanyl, sufentanil, alfentanil, remifentanil, levorphanol, dextromethorphan, phenazone, pemazocine, cyclazocine, methadone, isomethadone and propoxyphene. Suitable non-opioid analgesic agents include, but are not limited to, aspirin, celecoxib, rofecoxib, diclofenac, diflunisal, etodolac, fenoprofen, flurbiprofen, ibuprofen, ketoprofen, indomethacin, ketorolac, meclofenamate, mefenamic acid, nabumetone, naproxen, piroxicam and sulindac.

In yet another embodiment, the further therapeutic active agent can be an anxiolytic agent. Suitable anxiolytic agents include, but are not limited to, buspirone, and benzodiazepines such as diazepam, lorazepam, oxazapam, clorazepate, clonazepam, chlordiazepoxide and alprazolam.

The term "radiotherapeutic agent" as used herein, is intended to refer to any radiotherapeutic agent known to one of skill in the art to be effective to treat or ameliorate cancer, without limitation. For instance, the radiotherapeutic agent can be an agent such as those administered in brachytherapy or radionuclide therapy. Such methods can optionally further comprise the administration of one or more additional cancer therapies, such as, but not limited to, chemotherapies, and/or another radiotherapy.

Disclosed is a pharmaceutical composition comprising a polypeptide or nucleic acid and a pharmaceutically acceptable carrier. Typically, polypeptides or nucleic acids according to the invention may be combined with pharmaceutically acceptable excipients, and optionally sustained-release matrices, such as biodegradable polymers, to form therapeutic compositions.

"Pharmaceutically" or "pharmaceutically acceptable" refer to molecular entities and compositions that do not produce an adverse, allergic or other untoward reaction when administered to a subject, such as a human, as appropriate. A pharmaceutically acceptable carrier or excipient refers to a non-toxic solid, semi-solid or liquid filler, diluent, encapsulating material or formulation auxiliary of any type.

In the pharmaceutical compositions for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

The polypeptide may be formulated within a therapeutic mixture to comprise about 0.0001 to 1.0 milligrams, or about 0.001 to 0.1 milligrams, or about 0.1 to 1.0 milligrams, or about 1 to 10 milligrams or even about 10 to 100 milligrams per dose or so. Multiple doses can also be administered.

In addition to the compounds formulated for parenteral administration, such as intravenous or intramuscular injection, other pharmaceutically acceptable forms include, e.g. tablets or other solids for oral administration; liposomal formulations; time release capsules; and any other form currently used.

It is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope of the present invention. Any recited method can be carried out in the order of events recited or in any other order which is logically possible.

The invention is further described by the following non-limiting examples which further illustrate the invention, and are not intended, nor should they be interpreted to, limit the scope of the invention.

**EXAMPLE 1.** Activation of the MDA5-IPS1 viral sensing pathway induces cancer cell death and type I interferon-dependent antitumor immunity

### Abstract

Melanoma differentiation-associated gene 5 (MDA-5, IFIH1), a cytosolic innate pattern recognition receptor, functions as a first line of defense against viral infection by sensing double-stranded RNA (dsRNA). Ectopic expression of MDA-5 has been shown to induce cancer cell death, but the mechanism of action by which MDA-5 exerts these cytotoxic effects is unclear. Here, we demonstrate that ectopic expression of MDA-5 via replication incompetent adenovirus (Ad*.Mda-5*) initiates multiple signaling cascades, culminating in cytotoxicity and type I interferon (IFN) production in mouse and human prostate cancer cells. This intrinsic dual activity of MDA-5 required the adaptor protein IFN-β promoter stimulator 1 (IPS-1, MAV) and could be functionally uncoupled. MDA-5 lacking N-terminal caspase-recruitment domains (CARDs) engaged an intracellular death program in cancer cells, but was unable to efficiently stimulate expression of IFN-β. In contrast to cancer cells susceptible to MDA-5-mediated cytotoxicity, normal cells were highly resistant and instead developed a robust type I IFN response. Strikingly, intratumoral delivery of Ad*.Mda-5* led to regression of pre-established prostate cancers and development of long-lasting antitumor immune memory, which was primarily attributed to the activation of tumor-reactive cytotoxic T lymphocytes and/or natural killer cells. Using the CARDs-truncated MDA-5 mutant, silencing of IPS-1, and antibody blockade of the IFN-α/β-receptor, we further demonstrate that type I IFN signaling was crucial for in situ MDA-5-induced protective antitumor immunity. Therefore, deliberately targeting the evolutionarily conserved MDA-5-IPS-1 antiviral pathway in tumors can provoke parallel tumoricidal and immunostimulatory effects that bridge innate and adaptive immune responses for the therapeutic treatment of cancer.

### Introduction

Early work found that ectopic expression of *mda-5* gene induces death of cancer cells (12, 13). We and others recently showed that activation of MDA-5 *via* intracellular delivery of polyinosinic:polycytidylic acid or poly(I:C), a synthetic mimetic of viral dsRNA, stimulates intrinsic cell death program involving the pro-apoptotic molecules NOXA and Caspases in cancer cells, but not healthy cells (14-16). While MDA-5-induced susceptibility of cancer cells to apoptosis shares the features observed during the elimination of virus-infected cells upon MDA-5 activation, the underlying basis of MDA-5 action in engaging the cancer cell death pathway remains less understood. Given the critical role of MDA-5 in priming type I IFN response and antiviral immunity, the therapeutic potential of MDA-5 in enhancing immune activation against established tumors has yet to be determined.

### Materials and methods

### Mice

Male wild-type (WT) C57BL/6 mice were obtained from National Cancer Institute (Bethesda, MD). IPS-1-deficient mice (IPS-1^{-/-}), athymic nude mice were purchased from Jackson Laboratory (Bar Harbor, ME). Mice were maintained under specific pathogen-free conditions. All experiments and procedures involving mice were approved by the Institutional Animal Care and Use Committee of Virginia Commonwealth University.

### Cell lines and reagents

TRAMP-C2 cell line was derived from a prostate tumor that arose in a TRAMP (Transgenic Adenocarcinoma of Mouse Prostate) mouse on a C57BL/6 background (17, 18). TRAMP-C2 cells-expressing ovalbumin (C2-OVA) were generated in our laboratory (19). Human prostate adenocarcinoma line LNCaP and normal human prostate epithelial cell line RWPE-1 (CRL-11609) were from the American Type Culture Collection (ATCC). Cell line authentication was performed using short tandem repeat profiling DNA analysis and electrophoresis automatic sequencing by DDC Medical (Fairfield, OH). Mouse bone marrow-derived dendritic cells (BMDCs) were prepared as we previously described (20).

### Production of recombinant virus

Human *mda-5* (GenBank # NM_022168) and mouse *mda-5* (GenBank accession # NM_027835) cDNAs were obtained by RT-PCR from THP1-derived macrophages and poly(I:C)-stimulated BMDCs, respectively. The replication-deficient adenovirus (Ad) encoding human influenza hemagglutinin (HA)-tagged human MDA-5 (hMDA-5), mouse MDA-5 (mMDA-5), *CARD domains truncated forms of mouse or human MDA-5 (amino acid 252-1025,* mMDA-5HC or hMDA-5HC retaining the helicase and CTD domains), 1^{st} CARD domain truncated mouse MDA-5 (*amino acid 101-1025,* mMDA-5ΔC1), 2^{nd} CARD domain truncated mouse MDA-5 (*amino acid 101-200 deleted,* mMda-5ΔC2) and control virus Ad.*CMV* were constructed using AdEasy^{™} system and packaged in HEK293A cells as previously described (18). Lentiviruses (LV) encoding mouse IPS-1 shRNA for gene knockdown (LV-IPS-1/KD, GCAACCAGACTGGACCAAATA ; SEQ ID NO:5) and scrambled control shRNA (LV-SC) were packaged using Phoenix cells as previously described (21).

### Cell viability assays

Cell death was assessed using the Annexin V-FITC apoptosis detection kit (Roche Molecular Biochemicals, Germany) and analyzed by FACSCaliber flow cytometer (Becton Dickinson Biosciences, Franklin Lakes, NJ). Cell proliferation after Ad infection was measured using MTT assay as previously described (19).

### Tumor studies

TRAMP-C2 or C2-OVA tumor cells (2×10⁶) were injected subcutaneously (*s.c.*) in the right flank area of 6-8 weeks-old male C57BL/6 mice. 9-10 days after tumor implantation (approximately 3-5 mm in diameter), mice were randomly divided into groups and treated intratumorally (*i. t.*) with 1×10⁸ p.f.u. of Ads. in 50 µL of PBS every 3 days for a total of 5 doses. LNCaP cells (1×10⁷/mL) in 50 µL cold PBS were mixed with an equal volume of Matrigel and injected *s.c.* into the right flanks of male athymic nude mice. In some experiments, CD8⁺, CD4⁺ T cells, NK cells were depleted by intraperitoneal (*i.p.*) injection of 2.43, GK1.5, or PK136 antibodies, respectively, as described previously (18). For IFN-γ neutralization or blockade of type I IFN-α/β receptor, 200 µg anti-IFN-y (clone XMG1.2) or 1 mg anti-IFNARl (clone MAR1-5A3) antibodies from Bio X Cell (West Lebanon, NH) were administrated *i.p.* five times at 3-days intervals, beginning one day prior to therapy.

### Flow cytometric analysis and intracellular cytokine staining

Tumor tissues were processed by enzymatic digestion into single cell suspension as we previously described (18). Splenocytes from C2-OVA tumor bearing mice were stimulated with 1 µg/mL OVA₂₅₄₋₂₆₇ peptide for 4 days. Tumor-infiltrating leukocytes from tumor tissues, freshly isolated or peptide-stimulated splenocytes were stained with antibodies to CD8a, NK1.1, CD3, and analyzed with a flow cytometer. Intracellular staining of IFN-γ and Granzyme B was performed as previously described (22).

### qRT-PCR

Real time PCR was performed using TaqMan primers and carboxyfluorescein (FAM)-labeled probe sets from Life Technologies as we described previously (21). Target gene expression was normalized to *Bactin* and analyzed using the 2^{(-ΔΔCT)} relative quantification method.

### Cytotoxic activities of CTL and NK

The cytolytic activity of cytotoxic T cell (CTL) and natural killer (NK) cells was determined based on lactate dehydrogenase (LDH) release assay using a CytoTox 96 Non-Radioactive Cytotoxicity Assay Kit (Promega, Madison, WI).

### Statistical analysis

Experiments were repeated two or three times. Quantitative data are expressed as mean ± S.D. for all figure panels in which error bars are shown. Statistical significance between groups within experiments was determined by the Student's *t*-test or ANOVA test. A p value of less than 0.05 was considered statistically significant.

### Results

### Ectopic expression of MDA-5 triggers apoptosis and type I IFN production in prostate

### cancer cells

The proteolytic cleavage and separation of the N-terminal CARD domains from helicase domain of MDA-5 has been shown to accelerate apoptotic stimuli, such as virus infection and FasL treatment (11). To confirm the pro-apoptotic effect of ectopic expression of MDA-5, we infected mouse prostate cancer cells (i.e., TRAMP-C2) with an adenovirus encoding human MDA-5, mouse MDA-5, or mMDA-5HC (i.e., the CARD domains-truncated form of mouse MDA-5). Both mouse and human homologues of MDA-5 as well as mMDA-5HC were comparable in engaging intracellular death pathway, indicated by the cleavage of Caspase 3 and its main target poly (ADP-ribose) polymerase (PARP) (Fig 1A). Expressed full length MDA-5 appeared to undergo spontaneous MOI-dependent cleavage. Induction of cell death and growth inhibition of TRAMP-C2 tumor cells was confirmed by Annexin V staining (Figure 2A) and MTT assays (Fig. 1B), respectively. Infection of TRAMP-C2 cells with control *Ad.CMV* at a MOI up to 500 had no effect on cell viability. Consistent with an established role of MDA-5 in type I IFN response, overexpression of full length mMDA-5, not mMDA-5HC, strongly induced upregulation of IFN-β and IFN-α4 in TRAMP-C2 tumor cells (Fig. 1C). Similar results were obtained in human prostate cancer LNCaP cells upon infection with Ad*.hMda-5* or Ad*.hMda-5HC* (Fig. 1D-F, Figure 2B). These results indicate that ectopic expression of MDA-5 in cancer cells provokes a ligand-free, cell death signaling and type I IFN response, which share the similar features as previously reported in viral-infected cells. Moreover, the CARD domains are required for stimulation of type I IFN signaling, but dispensable for killing of cancer cells.

Taking into consideration that MDA-5 contains two N-terminal CARD domains, we constructed two additional truncated mouse MDA-5 mutants lacking either the first CARD (i.e., mMDA-5ΔC1) or the second CARD (i.e., mMDA-5ΔC2) to further dissect their roles in MDA-5-induced type I IFN response. As shown in Fig. 1G, absence of either CARD significantly reduced MDA-5 overexpression-triggered activation of the *Ifnb* gene in TRAMP-C2 cells. In agreement with analysis of mMDA-5HC, both mMDA-5ΔC1 and mMDA-5ΔC2 efficiently induced Caspase 3 activation and cancer cell death (Figure 2C-D).

### IPS-1 is required for MDA-5-induced cancer cell death and IFN-β production

Given the documented role of the adaptor protein IPS-1 in RIG-I-like receptor-induced type I IFN response (6), we examined the potential involvement of IPS-1 in MDA-5 expression-induced cancer cell death and type I IFN production. Lentiviral-mediated IPS-1 knockdown in TRAMP-C2 cells conferred substantial protection from MDA-5-induced cell death, as shown by reduced frequency of Annexin V⁺ cells (Fig. 3A) as well as decreased levels of the cleaved Caspase 3 or PARP (Fig. 3B).

The mitochondrial localization of IPS-1, disruption of mitochondria membrane potential, and cell death are important cellular responses to viral infection (23, 24). Confocal microscopy imaging showed a major cytoplasmic localization of ectopically expressed MDA-5 in Ad*.mMda-5* infected TRAMP-C2 cells. MDA-5 protein lacks putative mitochondrial targeting sequence based on conventional prediction algorithms (TargetP, MitoProt). However, a small portion of HA-tagged MDA-5 were clearly visible in the mitochondria compartment when co-staining with the mitochondrial marker MitoTracker (Fig. 3C), which suggests potential involvement of mitochondria-associated MDA-5 in induction of cell death. The active form of Caspase 3 has also been observed in the mitochondria, mostly translocated from the cytosol (25). Indeed, co-immunoprecipitation assays showed that ectopically expressed MDA-5 directly interacted with intracellular IPS-1, which appreciably increased IPS-1 association with Caspase 3 (Fig. 3D). Additionally, expression of full length MDA-5 or CARDs-truncated MDA-5 mutant caused similar enzymatic activation of Caspase 3 in TRAMP-C2 cells (Fig. 3E), which is consistent with their tumoricidal activities.

### IPS-1 mediates MDA-5 overexpression-induced type I IFN response

A Previous study reported that deficiency of IPS-1 abolished type I IFN response in various cell types upon viral infection (26). We found that genetically ablating IPS-1 in TRAMP-C2 tumor cells nearly completely blocked MDA-5 overexpression-induced IFN-β production. However, MDA-5HC failed to stimulate IFN-β production in TRAMP-C2 cells, suggesting the CARD domain is essential for MDA-5-IPS-1 signaling-triggered type I IFN response (Fig. 4A). Recruitment of transcription factor IRF3 to IPS-1 is required for its activation to coordinately regulate the type I IFNs during viral infection (5, 27). Immunoblotting analysis showed that MDA-5 overexpression in TRAMP-C2 induced the phosphorylation of IRF3, which was inhibited by IPS-1 knockdown, indicating that MDA-5-induced type I IFN production in cancer cells is dependent on IPS-1-IRF3 signaling pathway (Fig. 4B).

We next examined the effects of MDA-5 overexpression in dendritic cells (DCs), which play a pivotal role in responding to viral infection and in shaping host immunity (28). In contrast to cancer cells, ectopic expression of MDA-5 or MDA-5HC had little effect on viability of BMDCs (Fig. 5). However, both human and mouse MDA-5, but not CARDs-truncated MDA-5 (i.e., mMDA-5HC), induced high production of IFN-β (Fig. 4C). As expected, overexpression of either MDA-5 or MDA-5HC had no effect on PARP cleavage, while only full length MDA-5 was able to induce phosphorylation of IRF3 in BMDCs (Fig. 4D). Furthermore, MDA-5 overexpression-induced IFN-β production was substantially suppressed in either IPS-1 genetically ablated BMDCs (Fig. 4E) or IPS-1 deficient BMDCs (Fig. 4F), suggesting that MDA-5-initiated IPS-1-IRF3-type I IFN signaling pathway was identical and functional in both cancer cells and host myeloid cells.

Previous studies showed that intracellular delivery of synthetic dsRNA poly(I:C) using cationic liposome polyethylenimine (pIC-PEI) induces type I IFN-independent apoptosis in cancer cells *via* activation of MDA-5 (14-16). The concurrent upregulation of anti-apoptotic molecule Bcl-xL in normal cells is believed to render these cells less susceptible to pIC-PEI induced apoptosis (15). Similarly, primary BMDCs or normal human prostate epithelial cell line RWPE-1 were resistant to MDA-5 expression-induced apoptosis as compared with mouse (i.e., TRAMP-C2) or human (i.e., LNCaP) prostate cancer cells (Fig. 6A and 6B). We showed that pIC-PEI transfection only modestly induced MDA-5 expression when comparing with Ad*.mMda5,* however, pIC-PEI transfection enhanced the cleavage of PARP (Fig. 6A and 6B) in normal cells. This is further confirmed by Annexin V staining of BMDCs (Fig. 6C), indicating the onset of apoptosis in these normal cells following pIC-PEI treatment. Additionally, we did not see upregulation of Bcl-xL or MCL1 in pIC-PEI transfected normal cells (i.e., BMDCs. Fig. 6D) as previously reported (15).

Interestingly, ectopic expression of MDA-5 stimulated much higher levels of IRF3 activation in mouse TRAMP-C2 tumor cells than did pIC-PEI (Fig. 8A). Consistently, MDA-5 overexpression induced much higher magnitude and more sustained production of IFN-β as compared with pIC-PEI transfection (Fig. 8B). A similar enhanced IRF3 activation and IFN-β induction were also observed in Ad.*Mda*-*5*-infected BMDCs (Fig. 8C and 8D).

### In situ MDA-5 therapy potentiates an antitumor immune response to prostate cancer

To test the hypothesis that tumoricidal activity of MDA-5 and concurrent induction of type I IFNs drives antitumor immunity *in vivo,* we exploited OVA-expressing TRAMP-C2 tumor line (C2-OVA) that permits immunomonitoring of antigen-specific immune response (19). Mice established with C2-OVA tumors (average size 50 mm³) received *i.t.* treatment with Ad*.CMV,* Ad*.mMda-5,* and Ad.*mMda-5HC.* While Ad.*CMV* had little effect, Ad*.mMda-5HC* that is impaired in inducing type I IFNs partially delayed tumor growth. However, four out of five mice that had been treated with Ad*.mMda-5* rejected their tumors (Fig. 7A). TUNEL assays showed that Ad*.mMda-5* therapy caused significantly increased tumor cell death that positively correlated with enhanced tumor infiltration by NK1.1⁺ and CD8⁺ cells as compared with *Ad.CMV* or Ad*.mMda-5HC* (Fig. 10A). Ad*.mMda-5* treatment induced the highest levels of IFN-β, IFN-γ (Fig. 7B), MCP-1 and IL-12 (Fig. 10B) in the tumors, whereas Ad*.mMda-5HC* only resulted in moderate upregulation of IFN-γ expression. Flow cytometric analyses further confirmed the markedly increased frequencies of tumor-infiltrating NK cells and CD8⁺ T cells after MDA-5 therapy, which is consistent with the observation of high levels of MCP-1, a chemoattractant known to regulate the recruitment of T lymphocytes and NK cells (29, 30), as well as IL-12, which enhances the cytotoxic function of these antitumor effector cells (31). This elevated immune infiltration after Ad*.mMda-5* therapy was also associated with elevated activation of OVA-specific CD8⁺ T and NK cells, as determined by intracellular staining for IFN-γ (Fig. 7C) and Granzyme B (Fig. 10C).

### The therapeutic activity of MDA-5 depends on immune activation involving CD8⁺ T cells and NK cells

Upon stimulation with tumor cell lysates, splenocytes or draining lymph node cells from Ad*.mMda*-*5*-treated mice showed enhanced production of IFN-γ and IL-2 (Fig. 9A). The increased frequency of OVA-specific IFN-γ-producing CD8⁺ CTLs was similarly shown in the spleens from Ad*.mMda*-*5*-treated mice (Fig. 13A), suggesting systemic activation of tumor/antigen-reactive T cells by local MDA-5 therapy. Furthermore, splenocytes from mice receiving Ad*.mMda-5* displayed higher levels of cytotoxicity against the C2-OVA cells compared to those from Ad*.CMV* or Ad*.mMda-5HC-*treated mice (Fig. 9B). Enhanced cytolytic activity of NK cells, derived from Ad*.mMda*-*5*-treated mice, was also shown when either C2-OVA cells or YAC-1 cells were used as targets (Fig. 9C). To provide direct evidence and identify the immune cell subsets responsible for antitumor potency of MDA-5 therapy, we carried out *in vivo* depletion of leukocyte populations prior to treatment. Depletion of CD4⁺ cells modestly reduced the therapeutics effect, whereas the lack of CD8⁺ cells or NK cells ablated the antitumor activity of Ad*.mMda-5* (Fig. 9D).

In the C2-OVA tumor model, approximately 80 % of mice receiving *in situ* MDA-5 therapy showed tumor regression. These tumor-free mice were protected from tumor rechallenge two months later with either C2-OVA or parental TRAMP-C2 cells. Despite no tumor development in these mice upon secondary challenge, we showed strong induction of IFN-γ in the spleens or splenocytes stimulated with MHC I-restricted OVA₂₅₄₋₂₆₇ peptide (Fig. 13B and 13C), indicating the establishment of long-term protective immune memory.

### In situ MDA-5 therapy induces immune-mediated inhibition of human prostate cancer

Since NK cells are involved in the antitumor activity of MDA-5 therapy in immune competent mice, we further examined its potential role for controlling human prostate cancer (i.e., LNCaP) in athymic nude mice, which have a functional NK cell compartment. As predicted, Ad*.hMda-5* therapy induced NK cell activation, as shown by intracellular cytokine staining for IFN-γ-expression in tumor-infiltrating NK cells (Fig. 11A). These tumor-bearing mice, when depleted of NK cells, failed to respond to Ad*.hMda-5* treatment, resulting in outgrowth of human prostate cancer (Fig. 11B). Intriguingly, qRT-PCR analyses showed that both mouse and human forms of IFN-β were up-regulated in the LNCaP tumors, suggesting that administration of human MDA-5 elicits type I IFN response in human prostate cancer cells as well as in the stromal compartment and both of which may contribute to the functional activation of immune effector cells. Similar to the observation in mouse prostate tumor model, Ad*.hMda-5* also induced IFN-γ and MCP-1 production in the tumor site (Fig. 11C).

### Type I IFN pathway in the tumor sites is essential for antitumor efficacy of in situ MDA-5 therapy

Considering the robust induction of IFN-β in the tumor environment following MDA-5 treatment, we sought to define the contribution of type I IFN response to MDA-5 expression-generated antitumor immunity. *In vivo* blockade of IFN-β function using antibodies against IFN-α/β receptor subunit 1 (IFNAR-1) abolished the therapeutic effect of Ad*.mMda-5* treatment in C2-OVA tumor model, whereas neutralization of endogenous IFN-γ only partially attenuated the antitumor activity of MDA-5 (Fig. 12A). Antibody blockade of IFNAR-1 did not affect Ad*.mMda5*-induced IFN-β production, but significantly reduced the IFN-γ level in the tumor site (Fig. 14).

Considering that IPS-1 mediates type I IFN production by ectopic expression of MDA-5, we down-regulated the IPS-1 levels in the tumor sites by intra/peritumoral injection of lentivirus-encoding IPS-1 shRNA (Fig. 12B). IPS-1 silencing resulted in reduced production of both IFN-β and IFN-γ in the tumor sites (Fig. 12B), suggesting that IPS-1 is critical for Ad.*mMda-5*-induced type I IFN response and immune activation. As a result, IPS-1 silencing profoundly impaired the therapeutic efficacy of MDA-5 therapy (Fig. 12C).

### Discussion

The critical role of the innate PRR MDA-5 has been well recognized in the host antiviral response by sensing cytoplasmic dsRNA and initiating a well-synchronized signaling cascade resulting in upregulation of type I IFNs and an array of IFN-stimulated genes. Emerging evidence shows that ectopic expression of MDA-5 (2, 12, 13) or viral dsRNA mimetic (14-16, 32) directly induces a type I IFNs-independent tumoricidal effect in human melanoma cancer cells (15). Considering the therapeutic potential of MDA-5, the current study provides new insight into the molecular basis of MDA-5 function in provoking cancer cell death and type I IFN signaling. Moreover, we dissect the impact of these two features of MDA-5 on its therapeutic capacity in immune competent mouse model and demonstrate, for the first time, that MDA-5 activation elicited type I IFN signaling and consequent priming of innate and adaptive immune responses are essential for the antitumor potency of MDA-5-targeted therapy.

Our data show that ectopic expression of mouse or human MDA-5 in prostate cancer cells triggers two distinct and functionally independent pathways, i.e., induction of cell death and production of type I IFNs. Both mouse and human MDA-5 are comparable in engaging these two cellular processes, probably due to their sequence homology and evolutionarily conservative function in the host viral defense. Intriguingly, while tandem CARD domain-truncated MDA-5 (i.e., mouse or human MDA-5HC, mMDA-5ΔC1, or mMDA-5ΔC2) is fully competent in inducing cancer cell death, it lacks the ability to stimulate type I IFN production (Fig. 1), indicating that the CARDs are essential for MDA-5-initiated signaling to upregulate type I IFN genes (e.g., *Ifna4, Ifnb)* and, more importantly, these two functional features of MDA-5 may be uncoupled due to the cleavage and release of CARDs from MDA-5, which has been previously reported in viral-infected cells (9). Indeed, the CARDs were recently suggested to provide a polymerization-dependent signaling platform for recruiting the adaptor protein IPS-1 and amplifying the downstream signaling cascade (33). We also make an interesting finding that IPS-1 mediates MDA-5 overexpression induced Caspase 3-dependent cell death program as well as the induction of type I IFNs in cancer cells because silencing of IPS-1 impairs both pathways (Fig. 3). Confocal imaging and co-immunoprecipitation data indicate that at least a small portion of ectopically expressed MDA-5 resides in mitochondria organelle and is associated with IPS-1. Furthermore, MDA-5 expression in cancer cells clearly promotes the interaction of IPS-1 with Caspase 3, resulting in enhanced activation of Caspase 3.

We demonstrate that MDA-5 overexpression-induced cell death is specific for cancer cells (e.g., mouse or human prostate cancer), not normal cells (e.g., prostate epithelial cells, primary myeloid DCs), which is generally consistent with our previous finding in pIC-PEI - transfected human melanoma (14) and human pancreatic cancer (16) cells. However, ectopic expression of MDA-5 appears to more efficiently induce type I IFN production in both prostate cancer cells and normal cells. In addition, pIC-PEI transfection displays some cytotoxic effects on normal cells compared to adenovirus-mediated infection for MDA-5 overexpression that exhibits little normal cell toxicity, which may be caused by the activation of multiple PRRs (e.g., RIG-I, TLR3 or TLR5) in response to the pIC-PEI stimulation (34). Furthermore, compared to pIC-PEI, MDA-5 overexpression induces stronger activation of IRF3 as well as much more robust and sustained IFN-β production that may potentially support immune-mediated tumor destruction.

A striking finding of our study is that, in addition to its direct tumoricidal effect, tumor-targeted MDA-5 therapy potentiates highly potent antitumor immune responses and demonstrates superior antitumor activity (Fig. 7 and 9). *In situ* MDA-5 therapy using a non-replicative adenovirus (i.e., Ad*.mMda-5)* results in a cure of 80 % of syngeneic mouse prostate cancers established in immunocompetent mice. Generation of protective antitumor immunity is further indicated by the observation that these tumor-free mice resist secondary tumor challenge. *In situ* MDA-5 therapy-induced immune activation is also supported by elevation of type I IFN (i.e., IFN-β) and Th1 cytokine IFN-γ in the tumor microenvironment (TME) associated with increased frequencies in functionally activated, tumor-infiltrating cytotoxic CD8⁺ CTLs and NK cells. Furthermore, depletion of immune cell subsets reveals that both CD8⁺ T cells and NK cells are primarily responsible for the therapeutic inhibition of established TRAMP-C2 tumors, suggesting that mobilizing the innate and adaptive arms of the immune systems is a major antitumor mechanism underlying *in situ* MDA-5 therapy in immune competent mice.

Type I IFNs are critical for functional activation of NK cells and antigen-presenting cells (e.g., DCs) as well as cross-priming of CD8⁺ CTLs in immune surveillance or tumor immunotherapy (35-37). We have provided compelling evidence supporting a crucial role of type I IFN pathway in MDA-5 therapy-induced antitumor immunity. First, deletion of CARDs that are critical for type I IFN production in cancer cells substantially reduced the antitumor efficacy of MDA-5, which coincides with decreased activation and infiltration of cytotoxic effector cells in the TME (Fig. 7). Second, antibody blockade of receptor for type I IFNs attenuates the MDA-5 therapy-induced antitumor response (Fig. 12A). Lastly, disruption of MDA-5-triggered type I IFN production *via* silencing of IPS-1 in the TME also similarly abolished the antitumor effect of MDA-5 (Fig. 12C). Therefore, the intrinsic function of MDA-5 in stimulating the type I IFN pathway, compared to its pro-apoptotic activity in cancer cells, may be more essential in MDA-5 therapy-potentiated tumor eradication *in vivo,* at least in immune competent host (Fig. 12D). Given the ubiquitous expression of MDA-5 and induction of type I IFN in cancer cells as well as in the stroma after MDA-5 therapy, it is of particularly germane to understand the relative contribution of tumor or host cell-derived IPS-1-type I IFN pathway to overall antitumor immunity.

Collectively, we demonstrate that ectopic expression of MDA-5 selectively induces two distinct pathways, i.e., cancer cell death and type I IFN response, both of which is mediated by the adaptor molecule IPS-1. *In situ* MDA-5-targeted therapy generates superior antitumor immune response that is critically dependent on type I IFN response, highlighting a previously unappreciated role of MDA-5 in antitumor immunity. Since the immunosuppressive tumor microenvironment presents a major challenge for successful cancer immunotherapy, strategically engaging this ancient viral sensing MDA-5-IPS-1 pathway (38) in the TME represents a novel approach to efficiently reprograming or restoring protective antitumor immunity for cancer treatment.

### REFERENCES for EXAMPLE 1

1. Meylan E, Tschopp Jr, Karin M. Intracellular pattern recognition receptors in the host response. Nature. 2006;442:39-44.
2. Kang DC, Gopalkrishnan RV, Wu Q, Jankowsky E, Pyle AM, Fisher PB. mda-5: An interferon-inducible putative RNA helicase with double-stranded RNA-dependent ATPase activity and melanoma growth-suppressive properties. Proc Natl Acad Sci U S A. 2002;99:637-42.
3. Kato H, Takeuchi O, Sato S, Yoneyama M, Yamamoto M, Matsui K, et al. Differential roles of MDA5 and RIG-I helicases in the recognition of RNA viruses. Nature. 2006;441:101-5.
4. Wu B, Peisley A, Richards C, Yao H, Zeng X, Lin C, et al. Structural basis for dsRNA recognition, filament formation, and antiviral signal activation by MDA5. Cell. 2013;152:276-89.
5. Seth RB, Sun L, Ea CK, Chen ZJ. Identification and characterization of MAVS, a mitochondrial antiviral signaling protein that activates NF-kappaB and IRF 3. Cell. 2005;122:669-82.
6. Kawai T, Takahashi K, Sato S, Coban C, Kumar H, Kato H, et al. IPS-1, an adaptor triggering RIG-I- and Mda5-mediated type I interferon induction. Nature immunology. 2005;6:981-8.
7. Bowie AG, Unterholzner L. Viral evasion and subversion of pattern-recognition receptor signalling. Nat Rev Immunol. 2008;8:911-22.
8. Takeuchi O, Akira S. MDA5/RIG-I and virus recognition. Curr Opin Immunol. 2008;20:17-22.
9. Barral PM, Morrison JM, Drahos J, Gupta P, Sarkar D, Fisher PB, et al. MDA-5 is cleaved in poliovirus-infected cells. Journal of virology. 2007;81:3677-84.
10. Kuo RL, Kao LT, Lin SJ, Wang RY, Shih SR. MDA5 plays a crucial role in enterovirus 71 RNA-mediated IRF3 activation. PloS one. 2013;8:e63431.
11. Kovacsovics M, Martinon F, Micheau O, Bodmer JL, Hofmann K, Tschopp J. Overexpression of Helicard, a CARD-containing helicase cleaved during apoptosis, accelerates DNA degradation. Current biology : CB. 2002;12:838-43.
12. Kang DC, Gopalkrishnan RV, Lin L, Randolph A, Valerie K, Pestka S, et al. Expression analysis and genomic characterization of human melanoma differentiation associated gene-5, mda-5: a novel type I interferon-responsive apoptosis-inducing gene. Oncogene. 2004;23:1789-800.
13. Lin L, Su Z, Lebedeva IV, Gupta P, Boukerche H, Rai T, et al. Activation of Ras/Raf protects cells from melanoma differentiation-associated gene-5-induced apoptosis. Cell Death Differ. 2006;13:1982-93.
14. Tormo D, Checinska A, Alonso-Curbelo D, Pérez-Guijarro E, Cañón E, Riveiro-Falkenbach E, et al. Targeted Activation of Innate Immunity for Therapeutic Induction of Autophagy and Apoptosis in Melanoma Cells. Cancer Cell. 2009;16:103-14.
15. Besch R, Poeck H, Hohenauer T, Senft D, HÃ¤cker G, Berking C, et al. Proapoptotic signaling induced by RIG-I and MDA-5 results in type I interferonâ€"independent apoptosis in human melanoma cells. The Journal of Clinical Investigation. 2009;119:2399-411.
16. Bhoopathi P, Quinn BA, Gui Q, Shen XN, Grossman SR, Das SK, et al. Pancreatic cancer-specific cell death induced in vivo by cytoplasmic-delivered polyinosine-polycytidylic acid. Cancer Res. 2014;74:6224-35.
17. Foster BA, Gingrich JR, Kwon ED, Madias C, Greenberg NM. Characterization of prostatic epithelial cell lines derived from transgenic adenocarcinoma of the mouse prostate (TRAMP) model. Cancer Res. 1997;57:3325-30.
18. Yu X, Guo C, Yi H, Qian J, Fisher PB, Subjeck JR, et al. A multifunctional chimeric chaperone serves as a novel immune modulator inducing therapeutic antitumor immunity. Cancer research. 2013;73:2093-103.
19. Gao P, Sun X, Chen X, Wang Y, Foster BA, Subjeck J, et al. Secretable chaperone Grp170 enhances therapeutic activity of a novel tumor suppressor, mda-7/IL-24. Cancer Res. 2008;68:3890-8.
20. Yi H, Yu X, Gao P, Wang Y, Baek SH, Chen X, et al. Pattern recognition scavenger receptor SRA/CD204 down-regulates Toll-like receptor 4 signaling-dependent CD8 T-cell activation. Blood. 2009;113:5819-28.
21. Yu X, Yi H, Guo C, Zuo D, Wang Y, Kim HL, et al. Pattern recognition scavenger receptor CD204 attenuates Toll-like receptor 4-induced NF-kappaB activation by directly inhibiting ubiquitination of tumor necrosis factor (TNF) receptor-associated factor 6. The Journal of biological chemistry. 2011;286:18795-806.
22. Qian J, Yi H, Guo C, Yu X, Zuo D, Chen X, et al. CD204 suppresses large heat shock protein-facilitated priming of tumor antigen gp100-specific T cells and chaperone vaccine activity against mouse melanoma. J Immunol. 2011;187:2905-14.
23. Scott I, Norris KL. The mitochondrial antiviral signaling protein, MAVS, is cleaved during apoptosis. Biochemical and biophysical research communications. 2008;375:101-6.
24. Lei Y, Moore CB, Liesman RM, O'Connor BP, Bergstralh DT, Chen ZJ, et al. MAVS-mediated apoptosis and its inhibition by viral proteins. PloS one. 2009;4:e5466.
25. Chandra D, Tang DG. Mitochondrially localized active caspase-9 and caspase-3 result mostly from translocation from the cytosol and partly from caspase-mediated activation in the organelle. Lack of evidence for Apaf-1-mediated procaspase-9 activation in the mitochondria. The Journal of biological chemistry. 2003;278:17408-20.
26. Daffis S, Suthar MS, Szretter KJ, Gale M, Jr., Diamond MS. Induction of IFN-beta and the innate antiviral response in myeloid cells occurs through an IPS-1-dependent signal that does not require IRF-3 and IRF-7. PLoS pathogens. 2009;5:e1000607.
27. Liu S, Cai X, Wu J, Cong Q, Chen X, Li T, et al. Phosphorylation of innate immune adaptor proteins MAVS, STING, and TRIF induces IRF3 activation. Science. 2015;347:aaa2630.
28. Schmid MA, Diamond MS, Harris E. Dendritic cells in dengue virus infection: targets of virus replication and mediators of immunity. Frontiers in immunology. 2014;5:647.
29. Hamilton ST, Scott GM, Naing Z, Rawlinson WD. Human cytomegalovirus directly modulates expression of chemokine CCL2 (MCP-1) during viral replication. The Journal of general virology. 2013;94:2495-503.
30. Antonelli LR, Gigliotti Rothfuchs A, Goncalves R, Roffe E, Cheever AW, Bafica A, et al. Intranasal Poly-IC treatment exacerbates tuberculosis in mice through the pulmonary recruitment of a pathogen-permissive monocyte/macrophage population. The Journal of clinical investigation. 2010;120:1674-82.
31. Vignali DA, Kuchroo VK. IL-12 family cytokines: immunological playmakers. Nat Immunol. 2012;13:722-8.
32. Glas M, Coch C, Trageser D, Dassler J, Simon M, Koch P, et al. Targeting the cytosolic innate immune receptors RIG-I and MDA5 effectively counteracts cancer cell heterogeneity in glioblastoma. Stem cells. 2013;31:1064-74.
33. Berke IC, Yu X, Modis Y, Egelman EH. MDA5 assembles into a polar helical filament on dsRNA. Proceedings of the National Academy of Sciences of the United States of America. 2012;109:18437-41.
34. Cubillos-Ruiz JR, Engle X, Scarlett UK, Martinez D, Barber A, Elgueta R, et al. Polyethylenimine-based siRNA nanocomplexes reprogram tumor-associated dendritic cells via TLR5 to elicit therapeutic antitumor immunity. The Journal of Clinical Investigation. 2009;119:2231-44.
35. Swann JB, Hayakawa Y, Zerafa N, Sheehan KC, Scott B, Schreiber RD, et al. Type I IFN contributes to NK cell homeostasis, activation, and antitumor function. Journal of immunology. 2007;178:7540-9.
36. Dunn GP, Koebel CM, Schreiber RD. Interferons, immunity and cancer immunoediting. Nature reviews Immunology. 2006;6:836-48.
37. Diamond MS, Kinder M, Matsushita H, Mashayekhi M, Dunn GP, Archambault JM, et al. Type I interferon is selectively required by dendritic cells for immune rejection of tumors. J Exp Med. 2011;208:1989-2003.
38. Sarkar D, Desalle R, Fisher PB. Evolution of MDA-5/RIG-I-dependent innate immunity: independent evolution by domain grafting. Proc Natl Acad Sci U S A. 2008;105:17040-5.

### EXAMPLE 2. Combined effect of mda-5 and mda-7/IL-24 in B16 mouse melanoma xenograft studies.

Tumor xenografts were established subcutaneously in left flank of C57BL/6 mice by injecting 0.5^{∗}10⁶ cells of B16 cells mixed with matrigel in 1:1 ratio. Once tumors reached a palpable size, the mice were divided into different groups (four different groups and 3 mice for each group were used for survival and received virus either alone or in combination (10⁸vp for 8 doses). The mice were sacrificed as the tumor reached the maximum allowable limit. The survival curve was plotted which was calculated from the tumor plantation to the day of sacrifice. The day of sacrifice is the day when the tumor approaches the maximum allowable limit. As shown in Figure 16A, the *mda-5* and *mda-7* injected mice survive significantly more than other groups.

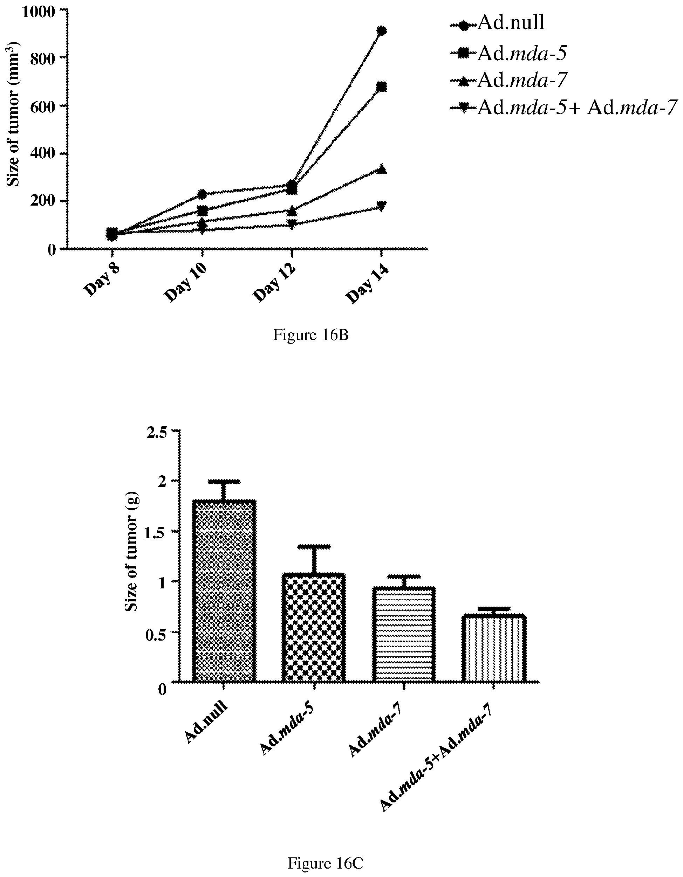

Tumor xenografts were established subcutaneously in the left flank of C57BL/6 mice by injecting 0.5^{∗}10⁶ cells of B16 cells mixed with matrigel in 1:1 ratio. Once tumors reached a palpable size, the mice were divided into different groups (four different groups and 3 mice per group) and received virus either alone or in combination (10⁸vp for 8 doses). The size of tumors were measured on each alternate day and the graph was plotted. As shown in Figure 16B, the *mda-5* and *mda-7* injected mice have smaller tumors than the mice injected with either *mda-5* or *mda-7* alone.

Tumor xenografts were established subcutaneously in left flank of C57BL/6 mice by injecting 0.5^{∗}10⁶ cells of B16 cells mixed with matrigel in 1:1 ratio. Once tumors reached a palpable size, the mice were divided into different groups (four different groups and 3 mice per group) and received virus either alone or in combination (10⁸vp for 8 doses). The weight of tumors were measured at the end point and the graph was plotted. As shown in Figure 16C, the *mda-5* and *mda-7* injected mice have smaller tumors than the mice injected with either *mda-5* or *mda-7* alone.

### EXAMPLE 3. Transgenic mouse model for prostate cancer.

A transgenic mouse model that develops prostate cancer was developed. A Hi-myc prostate tumor was isolated in culture (termed a murPDX). This tumor cell line was xenografted into Hi-myc mice and the tumor was infected with Ad.CTV. This caused tumor suppression in the infected xenograft as well as in the endogenous developing prostate tumor. As shown in Figure 17, there is a significant decrease in the xenograft tumor weight and the prostate weight.

### EXAMPLE 4. Combination of Ad.mda-5 with Ad.grp170 delivered by microbubble in the prostate of mice causes an inhibition in prostate cancer development.

Prostate cancer (CaP) is the most prevalent cancer and the second leading cause of cancer-related death in American men.¹ The therapeutic options for patients with prostate cancer include radiotherapy and treatment with cytotoxic chemotherapeutic agents. Despite a palliative benefit, these approaches do not engender a long-term beneficial effect on the overall survival of patients. Late stage prostate cancer patients may benefit from hormone therapy, or androgen deprivation therapy. Unfortunately, virtually all patients eventually progress to hormone-refractory disease after a short duration of response, resulting in therapy-resistant disease and patient death. Given that cancer is a highly heterogeneous disease resulting from genetic changes with histopathological variation, multi-functional strategies may be mandatory to promote improved treatment outcomes. One potential solution is to exploit novel therapeutic agents capable of beneficially and effectively promoting immunostimulatory prostate cancer death. A systemic and sustained antitumor immune response generated at the time of initial molecular-targeted therapy would provide significant clinical benefits in cancer patients.

The cytoplasmic RIG-I-like receptor MDA-5, a helicase now considered as a first line of defense against viral double-stranded RNA (dsRNA),² was initially identified as a melanoma differentiation-associated gene by our group.³ Upon recognition of viral dsRNA or its mimetic, e.g., poly(I:C), MDA-5 triggers a complex signal transduction cascade resulting in the activation of transcription factors, NF-κB and IFN regulatory factor 3 (IRF-3) in particular, and the production of cytokines including class I IFNs.^{4,5} Surprisingly, others and we revealed that ectopic expression of MDA-5 ⁶ or stimulation of MDA-5 ^{7,8} selectively led to induction of cell death in tumor cells, but not healthy cells. Therefore, 'danger' sensing MDA-5 represents a unique target for therapeutic intervention that may help overcome the characteristic resistance of prostate cancer to apoptosis.

Studies by others and us have demonstrated stress proteins are capable of integrating both innate and adaptive immune responses, and can be utilized as physiological adjuvants to develop novel immunotherapeutic approaches ⁹. Indeed, our extensive studies have shown that glucose-regulated protein 170 (grp170) is highly efficient in promoting antigen cross-presentation and tumor-specific immunity. ¹⁰⁻¹⁶ The immune-modulating activity of grp170 may be attributed to its highly efficient chaperoning capacity, because molecular chaperoning function is essential for the high potency of grp170 as an immune adjuvant. ¹³ To further improve the immunostimulatory activity of Grp170, We recently constructed a secretable grp170-flagellin hybrid chaperone (Flagrp170), which contains the defined NF-κB-activating region of flagellin. ¹⁷ Flagellin is a maj or structural protein of bacterial flagella ¹⁸ and a ligand for TLR5, ¹⁹ which has been shown to serve as a potent systemic adjuvant. ^{20,21} The control exerted by TLRs in linking innate and adaptive immunity is instrumental in the efficacy of vaccines containing TLR ligands. Many established and experimental vaccines incorporate agonists for TLRs, not only to protect against infectious diseases, but also in therapeutic immunization against cancer. ^{22,23} This engineered novel hybrid chaperone is designed not only to facilitate cross-presentation of associated tumor antigens (signal-1), but also to provide TLR-engaging signal (signal-2) crucial for the functional activation of DC.

Based on the unique activities of MDA-5 and Flagrp170, simultaneous targeting of tumor cells for apoptotic destruction (MDA-5) and immune compartment for activation of tumor immunity (Flagrp170) will be advantageous when combined into one therapeutic regimen, which will more effectively eradicate prostate cancer and/or micrometastatic diseases. The studies we proposed were designed to interrogate and evaluate a novel gene-based therapy targeting 'danger' sensing innate PRRs in both tumor and immune compartments. The synergistic actions of these two therapeutic agents provide a powerful and convenient mode for inducing systemic antitumor immunity; therefore, leading to the best long-term protection against prostate cancer. Our studies present evidence supporting this novel concept of tumor-site-targeted vaccination and provide an improved understanding of the underlying immunological mechanisms.

### Antitumor immune responses augmented by MDA-7/Flagrp170 treatment.

To test whether combined administration of Ad.MDA-5/Flagrp170 results in additive and potentially synergistic tumor control, we evaluated the antitumor efficacy of the combinatorial therapy in comparison with treatment with MDA-5 or Flagrp170 alone in the therapeutic setting. We showed that ccombinatorial delivery of Ad.MDA5/Flagrp170 resulted in a profound inhibition of the growth of established TRAMP-C2 tumor, when compared to single therapy with Ad.MDA-5 or Ad.Flagrp170 (Fig. 18A). Based on our recent work that intratumoral delivery of adenoviruses encoding Flagrp170 enhances the cross-presentation of tumor antigens and provokes a strong tumor-specific CTL response, ²⁷ we predicted that inclusion of Flagrp170 in the MDA-5-based treatment regimens would promote the cross-priming of antigen-specific T cells and consequently strengthening adaptive cellular immunity. Indeed, we showed that enhanced antitumor potency of the combinatorial therapy correlated with markedly enhanced production of IFN-γ or IL-2 in antigen (i.e., OVA as a model antigen)-stimulated splenocytes and lymph node cells (Fig. 18B).

### Antitumor immune responses augmented by MDA-5/Flagrp170 treatment.

As predicted, enhanced antitumor potency of the combinatorial therapy correlated with markedly increased activation of T lymphocytes. *In vitro* cytolytic assays indicate the involvement of both CD8⁺ cytotoxic T lymphocytes and cytotoxic NK cells in tumor destruction augmented by the combinatorial therapy. These findings support the hypothesis that the coordinated engagement of both innate and adaptive immune components contributes to the eradication of prostate cancer cells by the combinatorial thearpy. *In situ* Flagrp170 therapy preferentially drives the Th1 polarization of the tumor microenvironment (TME), as evidenced by marked elevation of IL-12 and IFN-γ ²⁷. We carried out experiments to determine the immune remodeling effect of the MDA-5/Flagrp170 combination therapy in the TME. We showed that Intratumoral administration of Ad.MDA-5/Flagrp170 resulted in the highest level of Th1 cytokines (IL-12 and IFN-y), CCL2 or chemoattractant monocyte chemoattractant protein-1 (MCP-1), and IFN-β in TRAMP-C2 prostate tumor tissues, as indicated by qRT-PCR (Fig. 19A). FACS analysis of tumor-infiltrating immune cells revealed that Ad.MDA5/Flagrp170 combination therapy preferentially increased the recruitment of IFN-γ-producing cytotoxic CD8⁺ T cells (Fig. 19B).

Based on highly potent immunostimulatory activity of Flagrp170 in enhancing the cross-presentation of tumor antigens and provoking a strong tumor-specific CTL response ²⁷, we predicted that combined MDA-5/Flagrp170 therapy results in additive and potentially synergistic antitumor effects. Our previous work showed that ccombinatorial delivery of Ad.MDA5/Flagrp170 led to a profound inhibition of established TRAMP-C2 tumors, compared to treatment with either Ad.MDA-5 or Ad.Flagrp170. Analyses of tumor infiltrating lymphocytes demonstrated that combined MDA-5/Flagrp170 regimen resulted in highly efficient recruitment of CD8⁺ T cells (i.e., CTLs) and NK cells (Fig. 20). These immune effector cells expressed high levels of cytotoxic molecules, such as IFN-γ or granzyme B, indicating Th1 polarization of the tumor microenvironment. Interestingly, our results also revealed differential immunostimulatory effects of Flagrp170 and MDA-5 in this combinatorial therapy protocol (Fig. 20). While MDA-5 preferientially stimulated the activation of NK cells, Flagrp170 enhanced an adaptive CTL response more efficiently (Fig. 20), which is in line with our findings on MDA-5 and Flagrp170 action in engaging a type I IFN response and antigen cross-presentation or T cell cross-priming ²⁷, respectively. Furthermore, FACS analysis of tumor infiltrating lymphocytes after MDA-5 and/or Flagrp170 identified the presence of CD4⁺CD8⁺ T cells with an effector memory phenotype. These CD4⁺CD8⁺ T cells have been shown to exhibit antiviral functions ³³ and suggested to participate in immune response to tumors in cancer patients ^{34,35}. It not clear how these tumor-associated CD4⁺CD8⁺ T cells may contribute to the therapeutic activity of MDA-5/Flagrp170 therapy. Analyses of tumor infiltrating lymphocytes following cancer treatment also reveal the distinct immunoregulatory effects of MDA-5 and Flagrp170, which is exemplified in the preferential activation of NK cells and T cells by these two unique therapeutic agents, respectively.

We also examined the effector functions of CTLs and NK cells using a different preclinical model (B16 tumor) after combined MDA-5/Flagrp170 therapy (Fig. 21). We showed that cytolytic activity of tumor-reactive T cells was substantially higher in tumor-bearing mice treated with MDA-5 plus Flagrp170 compared to those treated with single agent, i.e., MDA-5 or Flagrp170, (Fig. 21A), which positively correlates with enhanced tumor suppression or elimination by the combinatorial therapy. Similarly, the NK cells from MDA-5 plus Flagrp170-treated mice displayed the highest level of cytotoxicity against tumor cells (Fig. 21B) and NK-sensitive YAC-1 cells (data not shown). These molecular and cellular studies define the immunological responses induced by MDA-5 or Flagrp170, and provide mechanistic insights into the robust antitumor efficacy of the combinatorial MDA-5/Flagrp170 therapy.

### Evaluate the therapeutic efficacy of ultrasound-targeted microbubble assisted systemic delivery of Ad.mda-5/Flagrp170 to control spontaneous prostate tumors in the Hi-Myc-PEG-Luc (Hi-Myc-CCN1-Luc) transgenic mouse model.

We have investigated the feasibility of delivering MDA-5/Flagrp170 carrying adenoviruses using the ultrasound-targeted microbubble-destruction (UTMD) approach and evaluated the therapeutic potency of this combination in PC transgenic *Hi-myc* mice (Details of experimental protocol provided in the Figure legend) (Fig. 22A). Briefly ten four-month old *Hi-myc* mice were randomly divided into two groups (5 per group). Control group received decorated microbubble complexed with Ad. vec and the experimental group received therapeutic viruses (Ad*.Flgrp170* and Ad*.mda-5* at 1:1 ratio). A total of nine injections and UTMD were conducted within the first three weeks and all mice were sacrificed after 6-months of age. Tumors were weighed and a significant decrease in tumor weight (*p*<0.01) was evident in the therapeutic-treated group as compared to Ad.*vec*-treated group (Fig. 22B). H&E staining of sections prepared from the treated mice further confirmed the efficacy of the treatments. Invasive prostatic adenocarcinomas were observed in Ad.*vec*-treated 6-month old *Hi-myc* mice, whereas the invasive prostatic adenocarcinomas displayed decreased size in the Ad*.mda-5*/Ad*.Flagrp170* -treated group (Fig. 22C).

### Conclusion

Our research demonstrate that engineered chimeric chaperone Flagrp170 and MDA-5 represent two unique therapeutic agents for promoting therapeutic antitumor response. Rationally combined MDA-5/Flagrp170 treatment clearly shows therapeutic advantages over either single treatment, which we believe are attributed to the mobilization of multiple antitumor mechanisms, including direct cancer cell toxicity, immune activation and protective antitumor immunity. Mobilizing multivalent cancer-destroying pathways and establishing long-term protective immunity is anticipated to provide an optimal control of prostate cancer and metastases and to substantially reduce the risk of tumor escape. Our findings are expected to advance the field of cancer therapeutics, including those employing chemotherapy and immunotherapy, because our strategy offers a new approach to achieving *in situ* vaccination against the cancer and inducing antitumor immunity during initial molecular targeted therapy. The unique advantages of this rationally combined therapy provide significant benefits to prostate cancer patients. Moreover, our research on MDA-5 also revealed previously under-appreciated feature of this therapeutic molecule in promoting type I interferon response and innate immunity. This novel finding extends the previously reported role of MDA-5 in triggering cancer cell death and enhances our understanding of MDA-5 action as a therapeutic agent in engaging the host immune system. To facilitate the systemic delivery of Ad*.mda-5*/*Flagrp170* we are pioneering the use of targeted (decorated) microbubbles (d-MBs) combined with focused ultrasound, the ultrasound-targeted microbubble-destruction (UTMD) approach, to facilitate delivery of therapeutics adenoviruses in in target organs (e.g., prostate tumors) more efficiently. To evaluate our novel in situ cancer vaccination approach, we have successfully developed a unique double transgenic mouse model in which we can follow cancer progression in the prostate using non-invasive bioluminescence imaging approaches. Our findings provide a scientific rationale for combining molecular-targeted therapy with immunotherapies to achieve durable and optimal control of prostate cancer and metastasis.

### REFERENCES for EXAMPLE 4

1. Damber JE, A.G. Prostate cancer. Lancet 371, 1710-1721 (2008).
2. Kato, H., Takeuchi, O., Sato, S., Yoneyama, M., Yamamoto, M., Matsui, K., Uematsu, S., Jung, A., Kawai, T., Ishii, K.J., Yamaguchi, O., Otsu, K., Tsujimura, T., Koh, C.-S., Reis e Sousa, C., Matsuura, Y., Fujita, T. & Akira, S. Differential roles of MDA5 and RIG-I helicases in the recognition of RNA viruses. Nature 441, 101-105 (2006).
3. Kang, D.C., Gopalkrishnan, R.V., Wu, Q., Jankowsky, E., Pyle, A.M. & Fisher, P.B. mda-5: An interferon-inducible putative RNA helicase with double-stranded RNA-dependent ATPase activity and melanoma growth-suppressive properties. Proc Natl Acad Sci U S A 99, 637-642 (2002).
4. Bowie, A.G. & Unterholzner, L. Viral evasion and subversion of pattern-recognition receptor signalling. Nat Rev Immunol 8, 911-922 (2008).
5. Takeuchi, O. & Akira, S. MDA5/RIG-I and virus recognition. Curr Opin Immunol 20, 17-22 (2008).
6. Kang, D.C., Gopalkrishnan, R.V., Lin, L., Randolph, A., Valerie, K., Pestka, S. & Fisher, P.B. Expression analysis and genomic characterization of human melanoma differentiation associated gene-5, mda-5: a novel type I interferon-responsive apoptosis-inducing gene. Oncogene 23, 1789-1800 (2004).
7. Tormo, D., Checinska, A., Alonso-Curbelo, D., Perez-Guijarro, E., Canon, E., Riveiro-Falkenbach, E., Calvo, T.G., Larribere, L., Megias, D., Mulero, F., Piris, M.A., Dash, R., Barral, P.M., Rodriguez-Peralto, J.L., Ortiz-Romero, P., Tuting, T., Fisher, P.B. & Soengas, M.S. Targeted activation of innate immunity for therapeutic induction of autophagy and apoptosis in melanoma cells. Cancer Cell 16, 103-114 (2009).
8. Besch, R., Poeck, H., Hohenauer, T., Senft, D., Hacker, G., Berking, C., Hornung, V., Endres, S., Ruzicka, T., Rothenfusser, S. & Hartmann, G. Proapoptotic signaling induced by RIG-I and MDA-5 results in type I interferon-independent apoptosis in human melanoma cells. J Clin Invest 119, 2399-2411 (2009).
9. Wang, X.Y., Facciponte, J.G. & Subjeck, J.R. Molecular chaperones and cancer immunotherapy. Handb Exp Pharmacol, 305-329 (2006).
10. Wang, X.Y., Kazim, L., Repasky, E.A. & Subjeck, J.R. Characterization of heat shock protein 110 and glucose-regulated protein 170 as cancer vaccines and the effect of fever-range hyperthermia on vaccine activity. J Immunol 166, 490-497 (2001).
11. Park, J., Easton, D.P., Chen, X., MacDonald, I.J., Wang, X.Y. & Subjeck, J.R. The chaperoning properties of mouse grp170, a member of the third family of hsp70 related proteins. Biochemistry 42, 14893-14902 (2003).
12. Wang, X.Y., Arnouk, H., Chen, X., Kazim, L., Repasky, E.A. & Subjeck, J.R. Extracellular targeting of endoplasmic reticulum chaperone glucose-regulated protein 170 enhances tumor immunity to a poorly immunogenic melanoma. J Immunol 177, 1543-1551 (2006).
13. Park, J.E., Facciponte, J., Chen, X., MacDonald, I., Repasky, E.A., Manjili, M.H., Wang, X.Y. & Subjeck, J.R. Chaperoning function of stress protein grp170, a member of the hsp70 superfamily, is responsible for its immunoadjuvant activity. Cancer Research 66, 1161-1168 (2006).
14. Gao, P., Sun, X., Chen, X., Wang, Y., Foster, B.A., Subjeck, J., Fisher, P.B. & Wang, X.Y. Secretable chaperone Grp170 enhances therapeutic activity of a novel tumor suppressor, mda-7/IL-24. Cancer Res 68, 3890-3898 (2008).
15. Gao, P., Sun, X., Chen, X., Subjeck, J. & Wang, X.Y. Secretion of stress protein grp170 promotes immune-mediated inhibition of murine prostate tumor. Cancer Immunol Immunother 58, 1319-1328 (2009).
16. Wang, X.Y., Sun, X., Chen, X., Facciponte, J., Repasky, E.A., Kane, J. & Subjeck, J.R. Superior antitumor response induced by large stress protein chaperoned protein antigen compared with peptide antigen. J Immunol 184, 6309-6319 (2010).
17. Murthy, K.G., Deb, A., Goonesekera, S., Szabo, C. & Salzman, A.L. Identification of conserved domains in Salmonella muenchen flagellin that are essential for its ability to activate TLR5 and to induce an inflammatory response in vitro. J Biol Chem 279, 5667-5675 (2004).
18. Lowy, J. & Hanson, J. Structure of Bacterial Flagella. Nature 202, 538-540 (1964).
19. Hayashi, F., Smith, K.D., Ozinsky, A., Hawn, T.R., Yi, E.C., Goodlett, D.R., Eng, J.K., Akira, S., Underhill, D.M. & Aderem, A. The innate immune response to bacterial flagellin is mediated by Toll-like receptor 5. Nature 410, 1099-1103 (2001).
20. McSorley, S.J., Ehst, B.D., Yu, Y. & Gewirtz, A.T. Bacterial flagellin is an effective adjuvant for CD4+ T cells in vivo. J Immunol 169, 3914-3919 (2002).
21. Honko, A.N., Sriranganathan, N., Lees, C.J. & Mizel, S.B. Flagellin is an effective adjuvant for immunization against lethal respiratory challenge with Yersinia pestis. Infect Immun 74, 1113-1120 (2006).
22. Medzhitov, R. & Janeway, C.A., Jr. Decoding the patterns of self and nonself by the innate immune system. Science 296, 298-300 (2002).
23. van Duin, D., Medzhitov, R. & Shaw, A.C. Triggering TLR signaling in vaccination. Trends in Immunology 27, 49-55 (2006).
24. Andreakos, E., Williams, R.O., Wales, J., Foxwell, B.M. & Feldmann, M. Activation of NF-Î°B by the intracellular expression of NF-Î°B-inducing kinase acts as a powerful vaccine adjuvant. Proceedings of the National Academy of Sciences 103, 14459-14464 (2006).
25. Yu, X. & Wang, X.Y. Engineering Grp170-based immune modulators for cancer immunotherapy. Oncoimmunology 2, e24385 (2013).
26. Yu, X., Subjeck, J.R. & Wang, X.Y. Integrating a 'danger' signal into molecular chaperoning to improve vaccination against cancer. Expert Rev Vaccines 12, 581-583 (2013).
27. Yu, X., Guo, C., Yi, H., Qian, J., Fisher, P.B., Subjeck, J.R. & Wang, X.Y. A multifunctional chimeric chaperone serves as a novel immune modulator inducing therapeutic antitumor immunity. Cancer Res 73, 2093-2103 (2013).
28. Sun, Q., Sun, L., Liu, H.H., Chen, X., Seth, R.B., Forman, J. & Chen, Z.J. The specific and essential role of MAVS in antiviral innate immune responses. Immunity 24, 633-642 (2006).
29. Okamoto, M., Oshiumi, H., Azuma, M., Kato, N., Matsumoto, M. & Seya, T. IPS-1 is essential for type III IFN production by hepatocytes and dendritic cells in response to hepatitis C virus infection. Journal of immunology 192, 2770-2777 (2014).
30. Huang, Y., Liu, H., Li, S., Tang, Y., Wei, B., Yu, H. & Wang, C. MAVS-MKK7-JNK2 defines a novel apoptotic signaling pathway during viral infection. PLoS pathogens 10, e1004020 (2014).
31. Hamilton, S.T., Scott, G.M., Naing, Z. & Rawlinson, W.D. Human cytomegalovirus directly modulates expression of chemokine CCL2 (MCP-1) during viral replication. The Journal of general virology 94, 2495-2503 (2013).
32. Antonelli, L.R., Gigliotti Rothfuchs, A., Goncalves, R., Roffe, E., Cheever, A.W., Bafica, A., Salazar, A.M., Feng, C.G. & Sher, A. Intranasal Poly-IC treatment exacerbates tuberculosis in mice through the pulmonary recruitment of a pathogen-permissive monocyte/macrophage population. The Journal of clinical investigation 120, 1674-1682 (2010).
33. Nascimbeni, M., Shin, E.C., Chiriboga, L., Kleiner, D.E. & Rehermann, B. Peripheral CD4(+)CD8(+) T cells are differentiated effector memory cells with antiviral functions. Blood 104, 478-486 (2004).
34. Desfrancois, J., Moreau-Aubry, A., Vignard, V., Godet, Y., Khammari, A., Dreno, B., Jotereau, F. & Gervois, N. Double positive CD4CD8 alphabeta T cells: a new tumor-reactive population in human melanomas. PLoS One 5, e8437 (2010).
35. Desfrancois, J., Derre, L., Corvaisier, M., Le Mevel, B., Catros, V., Jotereau, F. & Gervois, N. Increased frequency of nonconventional double positive CD4CD8 alphabeta T cells in human breast pleural effusions. Int J Cancer 125, 374-380 (2009).
36. Dash, R., Azab, B., Quinn, B.A., Shen, X., Wang, X.Y., Das, S.K., Rahmani, M., Wei, J., Hedvat, M., Dent, P., Dmitriev, I.P., Curiel, D.T., Grant, S., Wu, B., Stebbins, J.L., Pellecchia, M., Reed, J.C., Sarkar, D. & Fisher, P.B. Apogossypol derivative BI-97C1 (Sabutoclax) targeting Mcl-1 sensitizes prostate cancer cells to mda-7/IL-24-mediated toxicity. Proc Natl Acad Sci USA 108, 8785-8790 (2011).
37. Mishra, A., Shiozawa, Y., Pienta, K.J. & Taichman, R.S. Homing of cancer cells to the bone. Cancer microenvironment : official journal of the International Cancer Microenvironment Society 4, 221-235 (2011).
38. Odero-Marah, V.A., Wang, R., Chu, G., Zayzafoon, M., Xu, J., Shi, C., Marshall, F.F., Zhau, H.E. & Chung, L.W. Receptor activator of NF-kappaB Ligand (RANKL) expression is associated with epithelial to mesenchymal transition in human prostate cancer cells. Cell research 18, 858-870 (2008).
39. Vaday, G.G., Hua, S.B., Peehl, D.M., Pauling, M.H., Lin, Y.H., Zhu, L., Lawrence, D.M., Foda, H.D. & Zucker, S. CXCR4 and CXCL12 (SDF-1) in prostate cancer: inhibitory effects of human single chain Fv antibodies. Clinical cancer research : an official journal of the American Association for Cancer Research 10, 5630-5639 (2004).
40. Ellwood-Yen, K., Graeber, T.G., Wongvipat, J., Iruela-Arispe, M.L., Zhang, J., Matusik, R., Thomas, G.V. & Sawyers, C.L. Myc-driven murine prostate cancer shares molecular features with human prostate tumors. Cancer Cell 4, 223-238 (2003).
41. Greco, A., Di Benedetto, A., Howard, C.M., Kelly, S., Nande, R., Dementieva, Y., Miranda, M., Brunetti, A., Salvatore, M., Claudio, L., Sarkar, D., Dent, P., Curiel, D.T., Fisher, P.B. & Claudio, P.P. Eradication of therapy-resistant human prostate tumors using an ultrasound-guided site-specific cancer terminator virus delivery approach. Molecular therapy : the journal of the American Society of Gene Therapy 18, 295-306 (2010).
42. Sarkar, S., Azab, B., Quinn, B.A., Shen, X., Dent, P., Klibanov, A.L., Emdad, L., Das, S.K., Sarkar, D. & Fisher, P.B. Chemoprevention gene therapy (CGT) of pancreatic cancer using perillyl alcohol and a novel chimeric serotype cancer terminator virus. Current molecular medicine 14, 125-140 (2014).
43. Anderson, C.R., Hu, X., Zhang, H., Tlaxca, J., Decleves, A.E., Houghtaling, R., Sharma, K., Lawrence, M., Ferrara, K.W. & Rychak, J.J. Ultrasound molecular imaging of tumor angiogenesis with an integrin targeted microbubble contrast agent. Investigative radiology 46, 215-224 (2011).
44. Ferrante, E.A., Pickard, J.E., Rychak, J., Klibanov, A. & Ley, K. Dual targeting improves microbubble contrast agent adhesion to VCAM-1 and P-selectin under flow. Journal of controlled release : official journal of the Controlled Release Society 140, 100-107 (2009).
45. Dash, R., Bhutia, S.K., Azab, B., Su, Z.Z., Quinn, B.A., Kegelmen, T.P., Das, S.K., Kim, K., Lee, S.G., Park, M.A., Yacoub, A., Rahmani, M., Emdad, L., Dmitriev, I.P., Wang, X.Y., Sarkar, D., Grant, S., Dent, P., Curiel, D.T. & Fisher, P.B. mda-7/IL-24: a unique member of the IL-10 gene family promoting cancer-targeted toxicity. Cytokine & growth factor reviews 21, 381-391 (2010).

While the invention has been described in its preferred embodiments, those of skill in the art will recognize the invention can be practiced with variations within the scope of the appended claims.

## Claims

1. A Melanoma Differentiation-Associated gene 5 (MDA-5)-encoding polynucleotide or a MDA-5 polypeptide, or a functional-conservative derivative thereof in a therapeutically effective amount for use in treating and preventing recurrence of cancer in a subject in need thereof.

2. The MDA-5-encoding polynucleotide or a MDA-5 polypeptide, or a functional conservative derivative thereof for use according to claim 1, wherein said cancer is prostate cancer.

3. The MDA-5-encoding polynucleotide or a MDA-5 polypeptide, or a functional conservative derivative thereof for use according to any one of the preceding claims, wherein said MDA-5 is a human or mouse MDA-5.

4. The MDA-5-encoding polynucleotide or a MDA-5 polypeptide, or a functional conservative derivative thereof for use according to any one of the preceding claims, wherein said MDA-5 includes one N-terminal caspase-recruitment domain (CARD) domain.

5. The MDA-5-encoding polynucleotide or a MDA-5 polypeptide, or a functional conservative derivative thereof for use according to any one of the preceding claims, comprising the use of the same by administering the same to a subject by directly contacting tumor cells of said cancer with said MDA-5-encoding polynucleotide or a MDA-5 polypeptide, or a functional conservative derivative thereof.

6. The MDA-5-encoding polynucleotide or a MDA-5 polypeptide, or a functional conservative derivative thereof for use according to any one of the preceding claims, comprising the step of administering the same to a subject by administering a vector selected from the group consisting of a plasmid, nanoparticle, cosmid, episome, artificial chromosome, phage and viral vector, in particular said viral vector is selected from the group consisting of adenoviral, retroviral, herpes virus, vaccinia virus, and an adeno-associated viral vector.

7. The MDA-5-encoding polynucleotide or a MDA-5 polypeptide, or a functional conservative derivative thereof for use according to any one of the preceding claims, further comprising a step of simultaneously or sequentially administering one or more active agents selected from the group consisting of a Melanoma Differentiation-Associated gene 7 (MDA-7)/interleukin-24 (IL-24)-encoding polynucleotide, a MDA-7/IL-24 polypeptide, and a functional-conservative derivative of said MDA-7/IL24.

8. The MDA-5-encoding polynucleotide or a MDA-5 polypeptide, or a functional conservative derivative thereof for use according to any one of the preceding claims, further comprising a step of simultaneously or sequentially administering one or more active agents selected from the group consisting of a glucose-regulated protein 170 (grp170), a grp170 polypeptide, and a functional-conservative derivative of said grp170.

9. The MDA-5-encoding polynucleotide or a MDA-5 polypeptide, or a functional conservative derivative thereof for use according to any one of the preceding claims, further comprising a step of simultaneously or sequentially administering one or more active agents selected from the group consisting of a grp170-flagellin hybrid (Flagrp170)-encoding polynucleotide, a Flagrp170 polypeptide, and a functional-conservative derivative of Flagrp170.

10. The MDA-5-encoding polynucleotide or a MDA-5 polypeptide, or a functional conservative derivative thereof for use according to any one of the preceding claims, further comprising a step of simultaneously or sequentially administering an immune checkpoint inhibitor.

11. The MDA-5-encoding polynucleotide or a MDA-5 polypeptide, or a functional conservative derivative thereof for use according to any one of the preceding claims, further comprising a step of simultaneously or sequentially administering one or more chemotherapeutic or radiotherapeutic agents.

12. The MDA-5-encoding polynucleotide or a MDA-5 polypeptide, or a functional conservative derivative thereof for use according to any one of the preceding claims, wherein said MDA-5-encoding polynucleotide is regulated by a cancer selective promoter.

13. The MDA-5-encoding polynucleotide or a MDA-5 polypeptide, or a functional conservative derivative thereof for use according to claim 12, wherein said cancer selective promoter is selected from the group consisting of progression elevated gene-3, astrocyte elevated gene-1, survivin, telomerase, truncated CCN1, and melanoma differentiation associated gene-9/syntenin-1.

14. A MDA-5-encoding polynucleotide or a MDA-5 polypeptide or a functional conservative derivative thereof in a therapeutically effective amount for use according to claim 1 in preventing recurrence of a cancer in a subject in remission.

15. The MDA-5-encoding polynucleotide or a MDA-5 polypeptide or a functional conservative derivative thereof for use according to claim 14 further comprising a step of simultaneously or sequentially administering one or more active agents selected from the group consisting of a MDA-7/IL-24-encoding polynucleotide, a MDA-7/IL-24 polypeptide, an immune checkpoint inhibitor, and a functional-conservative derivative thereof.

## Patentansprüche

1. Ein Melanom-Differenzierungs-Assoziiertes Gen 5 (Melanoma Differentiation-Associated gene, MDA-5) kodierendes Polynukleotid oder ein MDA-5-Polypeptid oder ein funktional konservatives Derivat davon, in einer therapeutisch wirksamen Menge zur Verwendung bei einer Behandlung oder zur Vorbeugung des Wiederauftretens von Krebs in einem dieses benötigenden Individuum.

2. Das MDA-5 kodierende Polynukleotid oder ein MDA-5 Polypeptid oder ein funktional konservatives Derivat davon zur Verwendung nach Anspruch 1, wobei dieser Krebs Prostatakrebs ist.

3. Das MDA-5-kodierende Polynukleotid oder ein MDA-5-Polypeptid oder ein funktional konservatives Derivat davon zur Verwendung nach einem der vorhergehenden Ansprüche, wobei das MDA-5 ein humanes oder Maus-MDA-5 ist.

4. Das MDA-5-kodierende Polynukleotid oder ein MDA-5-Polypeptid oder ein funktional konservatives Derivat davon zur Verwendung nach einem der vorhergehenden Ansprüche, wobei dieses MDA-5 eine N-terminale Caspase-Recruitment domain (CARD-Domäne) beinhaltet.

5. Das MDA-5-kodierende Polynukleotid oder ein MDA-5-Polypeptid oder ein funktional konservatives Derivat davon zur Verwendung nach einem der vorhergehenden Ansprüche, umfassend die Verwendung hiervon durch Verabreichung hiervon gegenüber einem Individuum durch direktes Inkontaktbringen der Tumorzellen dieses Krebses mit diesem MDA-5-kodierenden Polynukleotid oder einem MDA-5-Polypeptid oder einem funktionalen konservativen Derivat hiervon.

6. Das MDA-5 kodierende Polynukleotid oder ein MDA-5 Polypeptid oder ein funktional konservatives Derivat davon zur Verwendung nach einem der vorhergehenden Ansprüche, umfassend den Schritt des Verabreichens hiervon an ein Individuum durch Verabreichen eines Vektors, ausgewählt aus der Gruppe bestehend aus einem Plasmid, Nanopartikel, Cosmid, Episom, artifzielles Chromosom, Phage und viralem Vektor, insbesondere ist dieser virale Vektor ausgewählt aus der Gruppe bestehend aus adenoviralen, retroviralen, Herpesvirus, Vaccinia-Virus und einem adeno-assoziierten viralen Vektor.

7. Das MDA-5-kodierende Polynukleotid oder ein MDA-5-Polypeptid oder ein funktional konservatives Derivat hiervon zur Verwendung nach einem der vorhergehenden Ansprüche, weiterhin umfassend den Schritt des gleichzeitigen oder sequenziellen Verabreichens von einem oder mehreren wirksamen Mitteln, ausgewählt aus der Gruppe bestehend aus Melanom-Differenzierungs-assoziiertes Gen 7 (MDA-7)/lnterleukin-24 (IL-24)-kodierendes Polynukleotid, ein MDA-7/IL-24-Polypeptid und ein funktional-konservatives Derivat von MDA-7/IL-24.

8. Das MDA-5-kodierende Polynukleotid oder ein MDA-5-Polypeptid oder ein funktional konservatives Derivat hiervon zur Verwendung nach einem der vorhergehenden Ansprüche, weiterhin umfassend den Schritt des gleichzeitigen oder sequenziellen Verabreichens von einen oder mehreren wirksamen Mitteln, ausgewählt aus der Gruppe bestehend aus glukose-regulierten Protein 170 (grp170), einem grp170-Polypeptid, und einem funktional-konservativen Derivat des grp170.

9. Das MDA-5-kodierende Polynukleotid oder ein MDA-5-Polypeptid oder ein funktional konservatives Derivat hiervon zur Verwendung nach einem der vorgehenden Ansprüche, weiterhin umfassend den Schritt des gleichzeitigen oder sequenziellen Verabreichens von einem oder mehreren wirksamen Mitteln, ausgewählt aus der Gruppe bestehend aus grp170-Flagellin-Hybrid (Flagrp170)-kodierenden Polynukleotid, einem Flagrp170-Polypeptid und einem funktional konservativen Derivat von Flagrp170.

10. Das MDA-5-kodierende Polynukleotid oder ein MDA-5-Polypeptid oder ein funktional konservatives Derivat hiervon zur Verwendung nach einem der vorgehenden Ansprüche, weiterhin umfassend den Schritt des gleichzeitigen oder sequenziellen Verabreichens eines Immun-Checkpoint-Inhibitors.

11. Das MDA-5-kodierende Polynukleotid oder ein MDA-5-Polypeptid oder ein funktional konservatives Derivat hiervon zur Verwendung nach einem der vorgehenden Ansprüche, weiterhin umfassend den Schritt des gleichzeitigen oder sequenziellen Verabreichens von einer oder mehreren chemotherapeutischen oder radiotherapeutischen Mitteln.

12. Das MDA-5-kodierende Polynukleotid oder ein MDA-5-Polypeptid oder ein funktional konservatives Derivat hiervon zur Verwendung nach einem der vorhergehenden Ansprüche, wobei das MDA-5 kodierende Polynukleotid reguliert wird durch einen krebsselektiven Promotor.

13. Das MDA-5-kodierende Polynukleotid oder ein MDA-5-Polypeptid oder ein funktional konservatives Derivat davon zur Verwendung nach Anspruch 12, wobei diesee krebsselektive Promotor ausgewählt ist aus der Gruppe bestehend aus progressionserhöhendes Gen-3 (progression elevated gene-3), Astrozyt erhöhtes Gen- 1 (astrocyte elevated gene-1), Survivin, Telomerase, trunkierte CCN1, und Melanom-Differenzierungs-Assoziiertes Gen- 9/Syntenin-1.

14. Ein MDA-5-kodierendes Polynukleotid oder ein MDA-5-Polypeptid oder ein funktional konservatives Derivat hiervon in einer therapeutisch wirksamen Menge zur Verwendung nach Anspruch 1 zum Vorbeugen eines Wiederauftretens von Krebs in einem Individuum nach Remission.

15. Das MDA-5-kodierende Polynukleotid oder ein MDA-5-Polypeptid oder ein funktional konservatives Derivat davon zur Verwendung nach Anspruch 14 umfasst weiterhin den Schritt des gleichzeitigen oder sequenziellen Verabreichens von einem oder mehreren wirksamen Mitteln, ausgewählt aus der Gruppe bestehend aus MDA-7/IL-24-kodierendes Polynukleotid, ein MDA-7/IL-24-Polypeptid, ein Immun-Checkpoint-Inhibitor und einem funktional konservativen Derivat hiervon.

## Revendications

1. Polynucléotide codant pour le gène associé à la différenciation du mélanome 5 (MDA-5) ou polypeptide de MDA-5, ou dérivé conservateur fonctionnel de celui-ci en une quantité thérapeutiquement efficace pour utilisation dans le traitement et la prévention de la récidive de cancer chez un sujet en ayant besoin.

2. Polynucléotide codant pour MDA-5 ou polypeptide de MDA-5, ou dérivé conservateur fonctionnel de celui-ci pour utilisation selon la revendication 1, ledit cancer étant un cancer de la prostate.

3. Polynucléotide codant pour MDA-5 ou polypeptide de MDA-5, ou dérivé conservateur fonctionnel de celui-ci pour utilisation selon l'une quelconque des revendications précédentes, ledit MDA-5 étant un MDA-5 humain ou de souris.

4. Polynucléotide codant pour MDA-5 ou polypeptide de MDA-5, ou dérivé conservateur fonctionnel de celui-ci pour utilisation selon l'une quelconque des revendications précédentes, ledit MDA-5 comprenant un domaine de recrutement de caspase N-terminal (CARD).

5. Polynucléotide codant pour MDA-5 ou polypeptide de MDA-5, ou dérivé conservateur fonctionnel de celui-ci pour utilisation selon l'une quelconque des revendications précédentes, comprenant l'utilisation de celui-ci par administration de celui-ci à un sujet par mise en contact directe de cellules tumorales dudit cancer avec ledit polynucléotide codant pour MDA-5 ou un polypeptide de MDA-5, ou un dérivé conservateur fonctionnel de celui-ci.

6. Polynucléotide codant pour MDA-5 ou polypeptide de MDA-5, ou dérivé conservateur fonctionnel de celui-ci pour utilisation selon l'une quelconque des revendications précédentes, comprenant l'étape d'administration de celui-ci à un sujet par administration d'un vecteur choisi dans le groupe constitué d'un plasmide, d'une nanoparticule, d'un cosmide, d'un épisome, d'un chromosome artificiel, d'un phage et d'un vecteur viral, en particulier, ledit vecteur viral étant choisi dans le groupe constitué d'un vecteur viral adénoviral, rétroviral, de virus de l'herpès, de virus de la vaccine et adénoassocié.

7. Polynucléotide codant pour MDA-5 ou polypeptide de MDA-5, ou dérivé conservateur fonctionnel de celui-ci pour utilisation selon l'une quelconque des revendications précédentes, comprenant en outre une étape d'administration simultanée ou séquentielle d'un ou plusieurs agents actifs choisis dans le groupe constitué d'un polynucléotide codant pour le gène associé à la différenciation du mélanome 7 (MDA-7)/interleukine-24 (IL-24), d'un polypeptide de MDA-7/IL-24 et d'un dérivé conservateur fonctionnel dudit MDA-7/IL24.

8. Polynucléotide codant pour MDA-5 ou polypeptide de MDA-5, ou dérivé conservateur fonctionnel de celui-ci pour utilisation selon l'une quelconque des revendications précédentes, comprenant en outre une étape d'administration simultanée ou séquentielle d'un ou plusieurs agents actifs choisis dans le groupe constitué d'une protéine régulée par le glucose 170 (grp170), d'un polypeptide de grp170, et d'un dérivé conservateur fonctionnel dudit grp170.

9. Polynucléotide codant pour MDA-5 ou polypeptide de MDA-5, ou dérivé conservateur fonctionnel de celui-ci pour utilisation selon l'une quelconque des revendications précédentes, comprenant en outre une étape d'administration simultanée ou séquentielle d'un ou plusieurs agents actifs choisis dans le groupe constitué d'un polynucléotide codant pour un hybride grp170-flagelline (Flagrp170), d'un polypeptide de Flagrp170 et d'un dérivé conservateur fonctionnel de Flagrp170.

10. Polynucléotide codant pour MDA-5 ou polypeptide de MDA-5, ou dérivé conservateur fonctionnel de celui-ci pour utilisation selon l'une quelconque des revendications précédentes, comprenant en outre une étape d'administration simultanée ou séquentielle d'un inhibiteur de point de contrôle immunitaire.

11. Polynucléotide codant pour MDA-5 ou polypeptide de MDA-5, ou dérivé conservateur fonctionnel de celui-ci pour utilisation selon l'une quelconque des revendications précédentes, comprenant en outre une étape d'administration simultanée ou séquentielle d'un ou plusieurs agents chimiothérapeutiques ou radiothérapeutiques.

12. Polynucléotide codant pour MDA-5 ou polypeptide de MDA-5, ou dérivé conservateur fonctionnel de celui-ci pour utilisation selon l'une quelconque des revendications précédentes, ledit polynucléotide codant pour MDA-5 étant régulé par un promoteur sélectif pour un cancer.

13. Polynucléotide codant pour MDA-5 ou polypeptide de MDA-5, ou dérivé conservateur fonctionnel de celui-ci pour utilisation selon la revendication 12, ledit promoteur sélectif pour un cancer étant choisi dans le groupe constitué du gène élevé associé à une progression 3, du gène élevé dans les astrocytes 1, de la survivine, de la télomérase, de CCN1 tronqué et du gène associé à la différenciation du mélanome 9/synténine 1.

14. Polynucléotide codant pour MDA-5 ou polypeptide de MDA-5 ou dérivé conservateur fonctionnel de celui-ci en une quantité thérapeutiquement efficace pour utilisation selon la revendication 1 dans la prévention d'une récidive d'un cancer chez un sujet en rémission.

15. Polynucléotide codant pour MDA-5 ou polypeptide de MDA-5 ou un dérivé conservateur fonctionnel de celui-ci pour utilisation selon la revendication 14 comprenant en outre une étape d'administration simultanée ou séquentielle d'un ou plusieurs agents actifs choisis dans le groupe constitué d'un polynucléotide codant pour MDA-7/IL-24, d'un polypeptide MDA-7/IL-24, d'un inhibiteur de point de contrôle immunitaire et d'un dérivé conservateur fonctionnel de celui-ci.


### SEQUENCE LISTING

<110> VIRGINIA COMMONWEALTH UNIVERSITY
<120> TARGETING MDA-5 ACTIVATION FOR CANCER IMMUNOTHERAPY
<130> 02941073TA
<160> 9
<170> PatentIn version 3.5
<210> 1
   <211> 3617
   <212> DNA
   <213> Homo sapiens
<400> 1
   atcgaaacag aaaccaaagt caggcaaact ctgtaagaac tgcctgacag aaagctggac     60
   tcaaagctcc tacccgagtg tgcagcagga tcgccccggt ccgggacccc aggcgcacac    120
   cgcagagtcc aaagtgccgc gcctgccggc cgcacctgcc tgccgcggcc ccgcgcgccg    180
   ccccgctgcc cacctgcccg cctgcccacc tgcccaggtg cgagtgcagc cccgcgcgcc    240
   ggcctgagag ccctgtggac aacctcgtca ttgtcaggca cagagcggta gaccctgctt    300
   ctctaagtgg gcagcggaca gcggcacgca catttcacct gtcccgcaga caacagcacc    360
   atctgcttgg gagaaccctc tcccttctct gagaaagaaa gatgtcgaat gggtattcca    420
   cagacgagaa tttccgctat ctcatctcgt gcttcagggc cagggtgaaa atgtacatcc    480
   aggtggagcc tgtgctggac tacctgacct ttctgcctgc agaggtgaag gagcagattc    540
   agaggacagt cgccacctcc gggaacatgc aggcagttga actgctgctg agcaccttgg    600
   agaagggagt ctggcacctt ggttggactc gggaattcgt ggaggccctc cggagaaccg    660
   gcagccctct ggccgcccgc tacatgaacc ctgagctcac ggacttgccc tctccatcgt    720
   ttgagaacgc tcatgatgaa tatctccaac tgctgaacct ccttcagccc actctggtgg    780
   acaagcttct agttagagac gtcttggata agtgcatgga ggaggaactg ttgacaattg    840
   aagacagaaa ccggattgct gctgcagaaa acaatggaaa tgaatcaggt gtaagagagc    900
   tactaaaaag gattgtgcag aaagaaaact ggttctctgc atttctgaat gttcttcgtc    960
   aaacaggaaa caatgaactt gtccaagagt taacaggctc tgattgctca gaaagcaatg   1020
   cagagattga gaatttatca caagttgatg gtcctcaagt ggaagagcaa cttctttcaa   1080
   ccacagttca gccaaatctg gagaaggagg tctggggcat ggagaataac tcatcagaat   1140
   catcttttgc agattcttct gtagtttcag aatcagacac aagtttggca gaaggaagtg   1200
   tcagctgctt agatgaaagt cttggacata acagcaacat gggcagtgat tcaggcacca   1260
   tgggaagtga ttcagatgaa gagaatgtgg cagcaagagc atccccggag ccagaactcc   1320
   agctcaggcc ttaccaaatg gaagttgccc agccagcctt ggaagggaag aatatcatca     1380
   tctgcctccc tacagggagt ggaaaaacca gagtggctgt ttacattgcc aaggatcact 1440
   tagacaagaa gaaaaaagca tctgagcctg gaaaagttat agttcttgtc aataaggtac 1500
   tgctagttga acagctcttc cgcaaggagt tccaaccatt tttgaagaaa tggtatcgtg 1560
   ttattggatt aagtggtgat acccaactga aaatatcatt tccagaagtt gtcaagtcct 1620
   gtgatattat tatcagtaca gctcaaatcc ttgaaaactc cctcttaaac ttggaaaatg 1680
   gagaagatgc tggtgttcaa ttgtcagact tttccctcat tatcattgat gaatgtcatc 1740
   acaccaacaa agaagcagtg tataataaca tcatgaggca ttatttgatg cagaagttga 1800
   aaaacaatag actcaagaaa gaaaacaaac cagtgattcc ccttcctcag atactgggac 1860
   taacagcttc acctggtgtt ggaggggcca cgaagcaagc caaagctgaa gaacacattt 1920
   taaaactatg tgccaatctt gatgcattta ctattaaaac tgttaaagaa aaccttgatc 1980
   aactgaaaaa ccaaatacag gagccatgca agaagtttgc cattgcagat gcaaccagag 2040
   aagatccatt taaagagaaa cttctagaaa taatgacaag gattcaaact tattgtcaaa 2100
   tgagtccaat gtcagatttt ggaactcaac cctatgaaca atgggccatt caaatggaaa 2160
   aaaaagctgc aaaagaagga aatcgcaaag aacgtgtttg tgcagaacat ttgaggaagt 2220
   acaatgaggc cctacaaatt aatgacacaa ttcgaatgat agatgcgtat actcatcttg 2280
   aaactttcta taatgaagag aaagataaga agtttgcagt catagaagat gatagtgatg 2340
   agggtggtga tgatgagtat tgtgatggtg atgaagatga ggatgattta aagaaacctt 2400
   tgaaactgga tgaaacagat agatttctca tgactttatt ttttgaaaac aataaaatgt 2460
   tgaaaaggct ggctgaaaac ccagaatatg aaaatgaaaa gctgaccaaa ttaagaaata 2520
   ccataatgga gcaatatact aggactgagg aatcagcacg aggaataatc tttacaaaaa 2580
   cacgacagag tgcatatgcg ctttcccagt ggattactga aaatgaaaaa tttgctgaag 2640
   taggagtcaa agcccaccat ctgattggag ctggacacag cagtgagttc aaacccatga 2700
   cacagaatga acaaaaagaa gtcattagta aatttcgcac tggaaaaata aatctgctta 2760
   tcgctaccac agtggcagaa gaaggtctgg atattaaaga atgtaacatt gttatccgtt 2820
   atggtctcgt caccaatgaa atagccatgg tccaggcccg tggtcgagcc agagctgatg 2880
   agagcaccta cgtcctggtt gctcacagtg gttcaggagt tatcgaacat gagacagtta 2940
   atgatttccg agagaagatg atgtataaag ctatacattg tgttcaaaat atgaaaccag 3000
   aggagtatgc tcataagatt ttggaattac agatgcaaag tataatggaa aagaaaatga 3060
   aaaccaagag aaatattgcc aagcattaca agaataaccc atcactaata actttccttt 3120
   gcaaaaactg cagtgtgcta gcctgttctg ggaagatatc ccatgtaatt gagaaaatgc 3180
   atcacgtcaa tatgacccca gaattcaagg aactttacat tgtaagagaa aacaaagcac 3240
   tgcaaaagaa gtgtgccgac tatcaaataa atggtgaaat catctgcaaa tgtggccagg     3300
   cttggggaac aatgatggtg cacaaaggct tagatttgcc ttgtctcaaa ataaggaatt     3360
   ttgtagtggt tttcaaaaat aattcaacaa agaaacaata caaaaagtgg gtagaattac     3420
   ctatcacatt tcccaatctt gactattcag aatgctgttt atttagtgat gaggattagc     3480
   acttgattga agattctttt aaaatactat cagttaaaca tttaatatga ttatgattaa     3540
   tgtattcatt atgctacaga actgacataa gaatcaataa aatgattgtt ttactctgca     3600
   aaaaaaaaaa aaaaaaa                                                    3617
<210> 2
   <211> 1025
   <212> PRT
   <213> Homo sapiens
<400> 2
<210> 3
   <211> 5519
   <212> DNA
   <213> Mus musculus
<400> 3
   gttgacgtta ggaaagtctg gagaagagaa tcgaaacaga aaccgatctg tgggtggaag        60
   gcaatactta aagctccctg agggtgaacg tcccagcggc cgcagagcga gagcggtccc       120
   gggtctgtgc ccaccagttg cagcgctgtc ctggctcgtc tgccccctgc cgggtgccac       180
   cgatcgatcc ggtgctcgga ggctacttgg tacctgcggc tctcaggcag ttcaggcggg       240
   gacacattca ctgccacctg aggatcgtcc tcccgcccgc cccgcccgcc cgggagactc       300
   ctctcccatt tcctgagacc tcacgatgtc gattgtctgt tctgcagagg acagcttcag       360
   gaatctcatc ttattcttca ggcccaggct gaaaatgtac attcaggtgg agccagtgct       420
   ggaccacctc atctttctgt ctgcagaaac caaagagcag attcttaaaa agatcaacac       480
   ctgtggtaac accagcgcgg cagaactgct gctgagcacc ttggagcagg gacaatggcc       540
   tctgggatgg acgcagatgt tcgtggaggc cctagagcac agtggcaatc ccctagccgc       600
   gcgctatgtc aaacccacac tcactgatct gccctctcct tcctctgaga ctgcccatga       660
   cgagtgtctc cacttgctga ccctcctcca gcccactttg gtggacaaac ttctgattaa       720
   cgatgtcttg gacacttgct tcgagaaggg actattgaca gtcgaagaca gaaatcggat       780
   ttctgctgca ggaaacagcg ggaatgagtc aggtgtaaga gagctgctga gaaggattgt       840
   gcagaaggaa aactggtttt ctaccttcct ggatgttctg cgccaaactg gaaatgatgc 900
   actattccaa gaactaacag gtggaggctg cccagaagac aacacagact tggctaactc 960
   gtctcacaga gatgggcctg cagctaatga gtgtcttctg cctgctgtcg atgagtcaag 1020
   tctggagaca gaggcctgga acgtagacga catattacca gaggcttctt gtacagattc 1080
   gtctgtgacc acagaatcag acacaagttt ggcagaagga agtgtcagct gcttcgatga 1140
   aagtcttgga cataacagca acatgggcag ggattcaggc accatgggaa gtgattcaga 1200
   tgaaagtgtc atccagacaa aaagagtatc ccccgagcca gaactgcagc tcaggcctta 1260
   ccaaatggaa gtggcccaac cagctctaga tgggaagaat attattatct gcctccccac 1320
   ggggagtggg aaaaccagag tggctgttta catcaccaaa gatcacttag acaagaagaa 1380
   gcaggcatct gaatccggga aggttatcgt tcttgtcaat aaggtaatgt tagcagaaca 1440
   acttttccga aaagagttca acccatattt gaagaaatgg tatcgaatta ttggattaag 1500
   tggcgatacc cagctgaaaa tatcatttcc agaagttgtc aaatcttacg atgttattat 1560
   cagcactgct caaatccttg aaaactccct cttaaatctg gagagtggag acgatgacgg 1620
   tgtgcagctg tcagacttct ctctcattat cattgatgag tgccatcaca ccaacaagga 1680
   ggcagtctat aacaacatca tgagacgata tttgaagcag aagctgagaa acaatgacct 1740
   caagaaacaa aacaaaccag ccattcccct gccgcagata ctaggactga cagcttcacc 1800
   tggtgttgga gcagccaaaa agcagtctga ggctgaaaaa catattttaa atatatgtgc 1860
   caatcttgat gcctttacca ttaaaacagt gaaagagaat cttggtcaac tcaaacacca 1920
   aataaaggaa ccatgcaaga aatttgtgat tgctgatgac accagagaaa atccatttaa 1980
   agagaaactt ctagaaatta tggcaagcat tcagacttac tgccaaaaaa gtccaatgtc 2040
   agattttgga acccaacatt atgagcagtg ggccattcaa atggagaaaa aagctgctaa 2100
   agacggaaat cgcaaagatc gcgtctgtgc agaacatttg aggaagtaca acgaagccct 2160
   acaaatcaac gacacgatcc gaatgattga tgcatatagc cacctggaga cattctacac 2220
   tgatgagaaa gaaaagaagt tcgcagtcct caatgacagc gacaagagtg atgacgaggc 2280
   cagcagttgc aatgaccaac ttaagggcga tgtaaagaaa tctttgaaac tggacgaaac 2340
   ggatgaattt ctcatgaatt tgttctttga taacaagaaa atgttgaaaa aactagctga 2400
   aaacccaaaa tacgagaatg aaaaactcat taaattaaga aacacgatac tggaacaatt 2460
   cacaaggtct gaggagtcct cccgaggaat tatttttaca aaaacacgac agagcaccta 2520
   cgcactttcc cagtggatca tggaaaatgc aaagtttgcg gaagttggag tcaaagcgca 2580
   tcacctgatt ggcgcggggc acagcagtga agtcaagccc atgactcaga ctgaacaaaa 2640
   agaagtcatt agtaaatttc gcactggcga aataaatctg cttatcgcta cgacggtggc 2700
   agaggaaggc ctggatatca aagagtgcaa tattgttatt cgttatggcc ttgtcacgaa 2760
   cgagatagcc atggtccagg cccggggtcg agccagagct gatgaaagca cgtatgtcct 2820
   ggtcaccagc agtggctcag gagttaccga acgggagatt gttaatgatt tccgagagaa 2880
   gatgatgtat aaagctatta accgtgttca aaacatgaaa ccagaggagt atgcacataa 2940
   gattttggaa ttgcaggtgc aaagtatcct ggaaaagaaa atgaaagtca aaagaagcat 3000
   tgcaaagcaa tacaacgaca atccatcgtt aataacactt ctctgcaaaa attgtagcat 3060
   gctggtctgc tcgggagaaa acatccatgt cattgagaag atgcatcatg tcaatatgac 3120
   accagaattc aagggactct acattgtaag agaaaacaaa gcactgcaaa agaaatttgc 3180
   tgattatcag accaatggag agattatctg caagtgtggc caggcttggg gaacaatgat 3240
   ggtgcacaaa ggtttagatt tgccttgtct taaaataagg aattttgtag tcaatttcaa 3300
   aaataactca ccgaagaaac agtacaagaa gtgggtggaa ttgcctatca gatttcctga 3360
   tcttgactac tcagaatact gcttgtatag tgatgaagat tagcacttga ttcatgatta 3420
   ttttaaaata ttgccaactc aacattgaat atgattttaa ttgttgtttt cgctacacta 3480
   ctgagctaac tctagaatat gtagaggatg cttttgtttg gctttcagtg atgtaacaga 3540
   acaccaaaaa tgtaaacatc ttattggttg gtaaatgtaa ttggtaatca aaattgtcaa 3600
   tttgatgagt tctaaaatca cctgggctca gggaatgccc atgaggtatt gtcctaaatt 3660
   aattaaggaa gatctgttct ctgtgggtga tctcgggcta cataaaatta agaaaactag 3720
   ctgagtccac atttgctctc ataactttgc acttcattgt gcaatgcaat gtggcaagct 3780
   acttcaagct cctgtcactg tgactaccac acagtcatgg attaaaacct caaactgcaa 3840
   gctacaataa atcatttctt ccaaagttgt tatggtcagg gtgtttcatc acagcaacag 3900
   aaaagtaact aaaacatcag ggataaggaa aatctaccaa caatactcat aactgcaaag 3960
   tgttatagta ttatggaaac actggaatat aaatgagaac ccaagtacca atattagccc 4020
   tgtcactgat ttttttttcc agatctattt attttatgtc tatgtctgct tggcccgcat 4080
   gtatgtatgg gcaccatatg catttctggt gcctctggag gtcagagaaa ggcaccagat 4140
   cccctgaagt tggagttata gatgattgtg agccaccacg tgggggccgg gaatcaaaca 4200
   tgggtcctct gcatgagcaa caagtactca accactaaac cattgtccca gactctcggt 4260
   cactgatttc cacagtcaag ttcatctcct cagttcaccc ttcttccgca gcctccttat 4320
   ggtgaaatgt tgattgaact acctgcttct attgacaccc ttcagggttt taaatacacc 4380
   tacgcattat agcagctgtt ttgtatgcca aacatctaga tgtcgttttc ttacaaggct 4440
   gaaatcagtt tcagccagac tgttctgatc caaaggcctt gaaggaggaa acggtgctca 4500
   ctgacaatcc gtttccttgc agctgtctgg ctaggatttc tgttttcttg ctacatttcc 4560
   accaggaatt gctctcacat cctaggaatg ctttagcctg tggcttcttc aggaaggcct 4620
   cacagtacat atcatgtgtt tcttcaaggg cgagcagtag aaactctcaa agctactaag     4680
   gaatcttatg taacttaact atagtattag gagcacaata cattatgctt tggatgaggc     4740
   atcttatcga taagcagact tgaaacttac tctggattgt taactggtaa gattggcagt     4800
   gacctgggat aaggatgtct gggcattcct atcccgtgtg gctggtaggc catgcccaca     4860
   ctcaaagcaa ggggatatag ataattcagg atatcacgca tgaactgaca tgatttggga     4920
   aggaatcttg gaggtcattt tatagtcact tgttcatttt ttgctgttat gatgtatatc     4980
   cataggtgat catattgaca aacaggaaac tcgataaaga aagtaatgtt attaaaacac     5040
   agtatcaagt gggttcaggc ttgcttcttc tgccttctct tctgcaaaca cagtaccatc     5100
   ctggaatacg acctaatcat ctcttatgat gaagggagtg aagaaagtat tttttgtagt     5160
   tttttttttg tagtttaatt ccatattaag atatcttgaa tcttttattg atgagaactg     5220
   agccaaaatg aaaatattaa tcagaactct taggaatata aatggacaaa agaagaattg     5280
   gttttcttaa aatacttgaa gcattccatc taaatctatt taaagtattg ttctattcta     5340
   aagaattttg gaaactatgc aggaaatcaa atatctaaga tatccaagta gaccactgta     5400
   acatatatat tctgtttctg aaacatcaac aaatatcagg gtgtatattt gtttggtttt     5460
   gtcaatattt tatttcaata gttatttcta aattaaagat gtatatttca gatactatt      5519
<210> 4
   <211> 1025
   <212> PRT
   <213> Mus musculus
<400> 4
   Met Ser Ile Val Cys Ser Ala Glu Asp Ser Phe Arg Asn Leu Ile Leu
   1               5                   10                  15
   Phe Phe Arg Pro Arg Leu Lys Met Tyr Ile Gln Val Glu Pro Val Leu
               20                  25                  30
   Asp His Leu Ile Phe Leu Ser Ala Glu Thr Lys Glu Gln Ile Leu Lys
           35                  40                  45
   Lys Ile Asn Thr Cys Gly Asn Thr Ser Ala Ala Glu Leu Leu Leu Ser
       50                  55                  60
   Thr Leu Glu Gln Gly Gln Trp Pro Leu Gly Trp Thr Gln Met Phe Val
   65                  70                  75                  80
   Glu Ala Leu Glu His Ser Gly Asn Pro Leu Ala Ala Arg Tyr Val Lys
                   85                  90                  95
   Pro Thr Leu Thr Asp Leu Pro Ser Pro Ser Ser Glu Thr Ala His Asp
               100                 105                 110
   Glu Cys Leu His Leu Leu Thr Leu Leu Gln Pro Thr Leu Val Asp Lys
           115                 120                 125
   Leu Leu Ile Asn Asp Val Leu Asp Thr Cys Phe Glu Lys Gly Leu Leu
       130                 135                 140
   Thr Val Glu Asp Arg Asn Arg Ile Ser Ala Ala Gly Asn Ser Gly Asn
   145                 150                 155                 160
   Glu Ser Gly Val Arg Glu Leu Leu Arg Arg Ile Val Gln Lys Glu Asn
                   165                 170                 175
   Trp Phe Ser Thr Phe Leu Asp Val Leu Arg Gln Thr Gly Asn Asp Ala
               180                 185                 190
   Leu Phe Gln Glu Leu Thr Gly Gly Gly Cys Pro Glu Asp Asn Thr Asp
           195                 200                 205
   Leu Ala Asn Ser Ser His Arg Asp Gly Pro Ala Ala Asn Glu Cys Leu
       210                 215                 220
   Leu Pro Ala Val Asp Glu Ser Ser Leu Glu Thr Glu Ala Trp Asn Val
   225                 230                 235                 240
   Asp Asp Ile Leu Pro Glu Ala Ser Cys Thr Asp Ser Ser Val Thr Thr
                   245                 250                 255
   Glu Ser Asp Thr Ser Leu Ala Glu Gly Ser Val Ser Cys Phe Asp Glu
               260                 265                 270
   Ser Leu Gly His Asn Ser Asn Met Gly Arg Asp Ser Gly Thr Met Gly
           275                 280                 285
   Ser Asp Ser Asp Glu Ser Val Ile Gln Thr Lys Arg Val Ser Pro Glu
       290                 295                 300
   Pro Glu Leu Gln Leu Arg Pro Tyr Gln Met Glu Val Ala Gln Pro Ala
   305                 310                 315                 320
   Leu Asp Gly Lys Asn Ile Ile Ile Cys Leu Pro Thr Gly Ser Gly Lys
                   325                 330                 335
   Thr Arg Val Ala Val Tyr Ile Thr Lys Asp His Leu Asp Lys Lys Lys
               340                 345                 350
   Gln Ala Ser Glu Ser Gly Lys Val Ile Val Leu Val Asn Lys Val Met
           355                 360                 365
   Leu Ala Glu Gln Leu Phe Arg Lys Glu Phe Asn Pro Tyr Leu Lys Lys
       370                 375                 380
   Trp Tyr Arg Ile Ile Gly Leu Ser Gly Asp Thr Gln Leu Lys Ile Ser
   385                 390                 395                 400
   Phe Pro Glu Val Val Lys Ser Tyr Asp Val Ile Ile Ser Thr Ala Gln
                   405                 410                 415
   Ile Leu Glu Asn Ser Leu Leu Asn Leu Glu Ser Gly Asp Asp Asp Gly
               420                 425                 430
   Val Gln Leu Ser Asp Phe Ser Leu Ile Ile Ile Asp Glu Cys His His
           435                 440                 445
   Thr Asn Lys Glu Ala Val Tyr Asn Asn Ile Met Arg Arg Tyr Leu Lys
       450                 455                 460
   Gln Lys Leu Arg Asn Asn Asp Leu Lys Lys Gln Asn Lys Pro Ala Ile
   465                 470                 475                 480
   Pro Leu Pro Gln Ile Leu Gly Leu Thr Ala Ser Pro Gly Val Gly Ala
                   485                 490                 495
   Ala Lys Lys Gln Ser Glu Ala Glu Lys His Ile Leu Asn Ile Cys Ala
               500                 505                 510
   Asn Leu Asp Ala Phe Thr Ile Lys Thr Val Lys Glu Asn Leu Gly Gln
           515                 520                 525
   Leu Lys His Gln Ile Lys Glu Pro Cys Lys Lys Phe Val Ile Ala Asp
       530                 535                 540
   Asp Thr Arg Glu Asn Pro Phe Lys Glu Lys Leu Leu Glu Ile Met Ala
   545                 550                 555                 560
   Ser Ile Gln Thr Tyr Cys Gln Lys Ser Pro Met Ser Asp Phe Gly Thr
                   565                 570                 575
   Gln His Tyr Glu Gln Trp Ala Ile Gln Met Glu Lys Lys Ala Ala Lys
               580                 585                 590
   Asp Gly Asn Arg Lys Asp Arg Val Cys Ala Glu His Leu Arg Lys Tyr
           595                 600                 605
   Asn Glu Ala Leu Gln Ile Asn Asp Thr Ile Arg Met Ile Asp Ala Tyr
       610                 615                 620
   Ser His Leu Glu Thr Phe Tyr Thr Asp Glu Lys Glu Lys Lys Phe Ala
   625                 630                 635                 640
   Val Leu Asn Asp Ser Asp Lys Ser Asp Asp Glu Ala Ser Ser Cys Asn
                   645                 650                 655
   Asp Gln Leu Lys Gly Asp Val Lys Lys Ser Leu Lys Leu Asp Glu Thr
               660                 665                 670
   Asp Glu Phe Leu Met Asn Leu Phe Phe Asp Asn Lys Lys Met Leu Lys
           675                 680                 685
   Lys Leu Ala Glu Asn Pro Lys Tyr Glu Asn Glu Lys Leu Ile Lys Leu
       690                 695                 700
   Arg Asn Thr Ile Leu Glu Gln Phe Thr Arg Ser Glu Glu Ser Ser Arg
   705                 710                 715                 720
   Gly Ile Ile Phe Thr Lys Thr Arg Gln Ser Thr Tyr Ala Leu Ser Gln
                   725                 730                 735
   Trp Ile Met Glu Asn Ala Lys Phe Ala Glu Val Gly Val Lys Ala His
               740                 745                 750
   His Leu Ile Gly Ala Gly His Ser Ser Glu Val Lys Pro Met Thr Gln
           755                 760                 765
   Thr Glu Gln Lys Glu Val Ile Ser Lys Phe Arg Thr Gly Glu Ile Asn
       770                 775                 780
   Leu Leu Ile Ala Thr Thr Val Ala Glu Glu Gly Leu Asp Ile Lys Glu
   785                 790                 795                 800
   Cys Asn Ile Val Ile Arg Tyr Gly Leu Val Thr Asn Glu Ile Ala Met
                   805                 810                 815
   Val Gln Ala Arg Gly Arg Ala Arg Ala Asp Glu Ser Thr Tyr Val Leu
               820                 825                 830
   Val Thr Ser Ser Gly Ser Gly Val Thr Glu Arg Glu Ile Val Asn Asp
           835                 840                 845
   Phe Arg Glu Lys Met Met Tyr Lys Ala Ile Asn Arg Val Gln Asn Met
       850                 855                 860
   Lys Pro Glu Glu Tyr Ala His Lys Ile Leu Glu Leu Gln Val Gln Ser
   865                 870                 875                 880
   Ile Leu Glu Lys Lys Met Lys Val Lys Arg Ser Ile Ala Lys Gln Tyr
                   885                 890                 895
   Asn Asp Asn Pro Ser Leu Ile Thr Leu Leu Cys Lys Asn Cys Ser Met
               900                 905                 910
   Leu Val Cys Ser Gly Glu Asn Ile His Val Ile Glu Lys Met His His
           915                 920                 925
   Val Asn Met Thr Pro Glu Phe Lys Gly Leu Tyr Ile Val Arg Glu Asn
       930                 935                 940
   Lys Ala Leu Gln Lys Lys Phe Ala Asp Tyr Gln Thr Asn Gly Glu Ile
   945                 950                 955                 960
   Ile Cys Lys Cys Gly Gln Ala Trp Gly Thr Met Met Val His Lys Gly
                   965                 970                 975
   Leu Asp Leu Pro Cys Leu Lys Ile Arg Asn Phe Val Val Asn Phe Lys
               980                 985                 990
   Asn Asn Ser Pro Lys Lys Gln Tyr  Lys Lys Trp Val Glu  Leu Pro Ile
           995                 1000                1005
   Arg Phe  Pro Asp Leu Asp Tyr  Ser Glu Tyr Cys Leu  Tyr Ser Asp
       1010                1015                1020
   Glu Asp
       1025
<210> 5
   <211> 21
   <212> DNA
   <213> Artificial Sequence
<220>
   <223> Synthetic lentivirus encoding mouse IPS-1 shRNA
<400> 5
   gcaaccagac tggaccaaat a 21
<210> 6
   <211> 621
   <212> DNA
   <213> Homo sapiens
<400> 6
   atgaattttc aacagaggct gcaaagcctg tggactttag ccagaccctt ctgccctcct      60
   ttgctggcga cagcctctca aatgcagatg gttgtgctcc cttgcctggg ttttaccctg     120
   cttctctgga gccaggtatc aggggcccag ggccaagaat tccactttgg gccctgccaa     180
   gtgaaggggg ttgttcccca gaaactgtgg gaagccttct gggctgtgaa agacactatg     240
   caagctcagg ataacatcac gagtgcccgg ctgctgcagc aggaggttct gcagaacgtc     300
   tcggatgctg agagctgtta ccttgtccac accctgctgg agttctactt gaaaactgtt     360
   ttcaaaaact accacaatag aacagttgaa gtcaggactc tgaagtcatt ctctactctg     420
   gccaacaact ttgttctcat cgtgtcacaa ctgcaaccca gtcaagaaaa tgagatgttt     480
   tccatcagag acagtgcaca caggcggttt ctgctattcc ggagagcatt caaacagttg     540
   gacgtagaag cagctctgac caaagccctt ggggaagtgg acattcttct gacctggatg     600
   cagaaattct acaagctctg a                                               621
<210> 7
   <211> 206
   <212> PRT
   <213> Homo sapiens
<400> 7
   Met Asn Phe Gln Gln Arg Leu Gln Ser Leu Trp Thr Leu Ala Arg Pro
   1               5                   10                  15
   Phe Cys Pro Pro Leu Leu Ala Thr Ala Ser Gln Met Gln Met Val Val
               20                  25                  30
   Leu Pro Cys Leu Gly Phe Thr Leu Leu Leu Trp Ser Gln Val Ser Gly
           35                  40                  45
   Ala Gln Gly Gln Glu Phe His Phe Gly Pro Cys Gln Val Lys Gly Val
       50                  55                  60
   Val Pro Gln Lys Leu Trp Glu Ala Phe Trp Ala Val Lys Asp Thr Met
   65                  70                  75                  80
   Gln Ala Gln Asp Asn Ile Thr Ser Ala Arg Leu Leu Gln Gln Glu Val
                   85                  90                  95
   Leu Gln Asn Val Ser Asp Ala Glu Ser Cys Tyr Leu Val His Thr Leu
               100                 105                 110
   Leu Glu Phe Tyr Leu Lys Thr Val Phe Lys Asn Tyr His Asn Arg Thr
           115                 120                 125
   Val Glu Val Arg Thr Leu Lys Ser Phe Ser Thr Leu Ala Asn Asn Phe
       130                 135                 140
   Val Leu Ile Val Ser Gln Leu Gln Pro Ser Gln Glu Asn Glu Met Phe
   145                 150                 155                 160
   Ser Ile Arg Asp Ser Ala His Arg Arg Phe Leu Leu Phe Arg Arg Ala
                   165                 170                 175
   Phe Lys Gln Leu Asp Val Glu Ala Ala Leu Thr Lys Ala Leu Gly Glu
               180                 185                 190
   Val Asp Ile Leu Leu Thr Trp Met Gln Lys Phe Tyr Lys Leu
           195                 200                 205
<210> 8
   <211> 312
   <212> DNA
   <213> Homo sapiens
<400> 8
   gagagctgtt accttgtcca caccctgctg gagttctact tgaaaactgt tttcaaaaac      60
   taccacaata gaacagttga agtcaggact ctgaagtcat tctctactct ggccaacaac     120
   tttgttctca tcgtgtcaca actgcaaccc agtcaagaaa atgagatgtt ttccatcaga     180
   gacagtgcac acaggcggtt tctgctattc cggagagcat tcaaacagtt ggacgtagaa     240
   gcagctctga ccaaagccct tggggaagtg gacattcttc tgacctggat gcagaaattc     300
   tacaagctct ga                                                         312
<210> 9
   <211> 103
   <212> PRT
   <213> Homo sapiens
<400> 9
   Glu Ser Cys Tyr Leu Val His Thr Leu Leu Glu Phe Tyr Leu Lys Thr
   1               5                   10                  15
   Val Phe Lys Asn Tyr His Asn Arg Thr Val Glu Val Arg Thr Leu Lys
               20                  25                  30
   Ser Phe Ser Thr Leu Ala Asn Asn Phe Val Leu Ile Val Ser Gln Leu
           35                  40                  45
   Gln Pro Ser Gln Glu Asn Glu Met Phe Ser Ile Arg Asp Ser Ala His
       50                  55                  60
   Arg Arg Phe Leu Leu Phe Arg Arg Ala Phe Lys Gln Leu Asp Val Glu
   65                  70                  75                  80
   Ala Ala Leu Thr Lys Ala Leu Gly Glu Val Asp Ile Leu Leu Thr Trp
                   85                  90                  95
   Met Gln Lys Phe Tyr Lys Leu
               100